(19) Europäisches Patentamt
European Patent Office
Office européen des brevets

(11) EP 4 318 104 A1

(12) EUROPEAN PATENT APPLICATION
published in accordance with Art. 153(4) EPC

(43) Date of publication:
**07.02.2024 Bulletin 2024/06**

(21) Application number: **22780135.4**

(22) Date of filing: **16.03.2022**

(51) International Patent Classification (IPC):
***G02C 7/10*** *(2006.01)* ***A61B 5/0245*** *(2006.01)*
***G02B 1/04*** *(2006.01)* ***G02B 5/22*** *(2006.01)*

(52) Cooperative Patent Classification (CPC):
**A61B 5/0245; G02B 1/04; G02B 5/22; G02C 7/10**

(86) International application number:
**PCT/JP2022/012027**

(87) International publication number:
**WO 2022/209915 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2021 JP 2021060675**

(71) Applicant: **MITSUI CHEMICALS, INC.**
**Tokyo 104-0028 (JP)**

(72) Inventors:
- **TAKENAKA, Manami**
  **Omuta-shi, Fukuoka 836-8610 (JP)**
- **KAWATO, Nobuo**
  **Omuta-shi, Fukuoka 836-8610 (JP)**

(74) Representative: **Mewburn Ellis LLP**
**Aurora Building**
**Counterslip**
**Bristol BS1 6BX (GB)**

# (54) OPTICAL ELEMENT, GLASSES LENS, AUTONOMIC NERVE REGULATION METHOD, AND EVALUATION METHOD FOR OPTICAL ELEMENT

(57) An optical element, in which Stim (ipRGC) represented by Formula 1 is from 10.0 to 68.0. In Formula 1, $RI_{D65}$ (λ) is a spectral irradiance of illuminant D65, τ (λ) is a spectral transmittance of the optical element, and $S_{mel}$ (λ) is a sensitivity function of ipRGCs obtained by converting a sensitivity function of ipRGCs described in CIE S/026E: 2018 such that a maximum sensitivity thereof is identical to a maximum sensitivity of a z bar (λ) in CIE 1931 color-matching functions.

$$\mathrm{Stim(ipRGC)} = \frac{\int_{390nm}^{780nm} \{RI_{D65}(\lambda) \cdot \tau(\lambda) \cdot S_{mel}(\lambda)\} d\lambda}{\int_{390nm}^{780nm} \{RI_{D65}(\lambda) \cdot S_{mel}(\lambda)\} d\lambda} \quad \text{Formula 1}$$

EP 4 318 104 A1

FIG.1

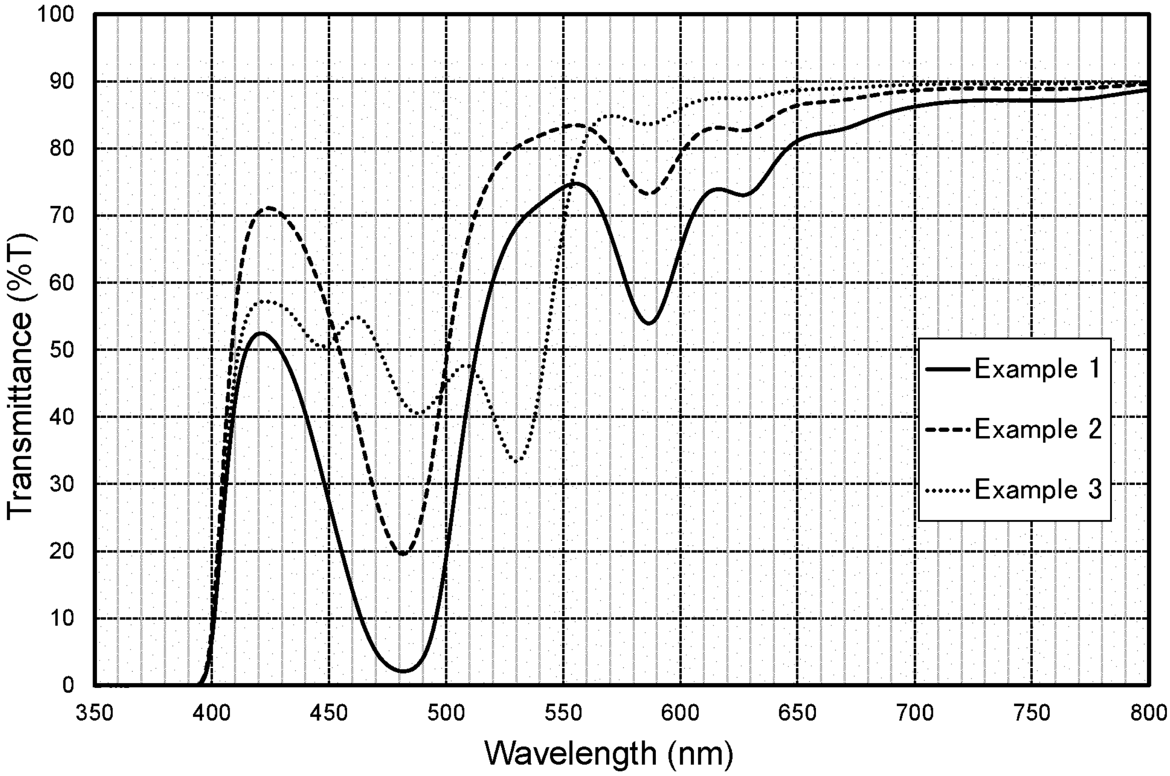
100
90
80
70
60
50
40
30
20
10
0
350
400
450
500
550
600
650
700
750
800
Transmittance (%T)
Wavelength (nm)
Example 1
Example 2
Example 3

## Description

### TECHNICAL FIELD

The present disclosure relates to an optical element, an eyeglass lens, an autonomic nerve regulation method, and an evaluation method for an optical element.

### BACKGROUND ART

Plastic lenses are lighter than inorganic lenses, are less likely to break, and can be dyed. Therefore, the plastic lenses have rapidly become widespread in optical materials such as eyeglass lenses and camera lenses.

In recent years, an eyeglass lens that reduces the influence on the eyes by suppressing the transmission of light having a specific wavelength has been developed.

Patent Document 1 describes an optical material in which a transmittance curve measured at a thickness of 2 mm satisfies the following characteristics (1) and a luminous transmittance satisfies the following characteristics (2).

(1) A maximum transmittance at a wavelength of from 410 nm to 450 nm is from 20% to 65%.
(2) A luminous transmittance is from 70% to 85%.

Patent Document 1: International Publication No. 2020/213717

### SUMMARY OF INVENTION

#### Technical Problem

As photoreceptors, rods, cones (S cones, M cones, L cones), and intrinsically photosensitive retinal ganglion cells (ipRGCs) are present on the retina of mammals.

It has been suggested that these photoreceptors may be involved in, for example, the symptoms of migraine.

The present inventors have conducted various studies and considerations regarding the control of light incident on a photoreceptor, and have conducted intensive studies. As a result, the inventors have found that there is a possibility that autonomic nerves can be affected by controlling the light incident on the photoreceptor.

As a result of further research based on the above findings, the inventors have completed an eyeglass lens that regulates the autonomic nerves by controlling the light incident on the photoreceptor.

Patent Document 1 does not consider regulating the autonomic nerves by controlling the light incident on the photoreceptor.

An object to be achieved by an embodiment of the disclosure is to provide an optical element capable of regulating autonomic nerves, an eyeglass lens, an autonomic nerve regulation method, and an evaluation method for an optical element.

#### Solution to Problem

Specific means for achieving the above object include the following embodiments.

<1> An optical element, in which Stim (ipRGC) represented by the following Formula 1 is from 10.0 to 68.0:

$$\mathrm{Stim(ipRGC)} = \frac{\int_{390nm}^{780nm} \{RI_{D65}(\lambda) \cdot \tau(\lambda) \cdot S_{mel}(\lambda)\} d\lambda}{\int_{390nm}^{780nm} \{RI_{D65}(\lambda) \cdot S_{mel}(\lambda)\} d\lambda} \qquad \text{Formula 1}$$

wherein, in Formula 1, $RI_{D65}$ (λ) is a spectral irradiance of illuminant D65, τ (λ) is a spectral transmittance of the optical element, and $S_{mel}$ (λ) is a sensitivity function of ipRGCs obtained by converting a sensitivity function of ipRGCs described in CIE S/026E: 2018 such that a maximum sensitivity thereof is identical to a maximum sensitivity of a z bar (λ) in CIE 1931 color-matching functions.

<2> The optical element according to <1>, wherein a sum of the Stim (ipRGC) and Stim (L) represented by the following Formula 2 is from 20.0 to 135.0:

$$\text{Stim}(L) = \frac{\int_{390nm}^{780nm} \{RI_{D65}(\lambda) \cdot \tau(\lambda) \cdot S_L(\lambda)\} d\lambda}{\int_{390nm}^{780nm} \{RI_{D65}(\lambda) \cdot S_L(\lambda)\} d\lambda} \quad \text{Formula 2}$$

wherein, in Formula 2, $RI_{D65}$ ($\lambda$) is the spectral irradiance of illuminant D65, $\tau$ ($\lambda$) is the spectral transmittance of the optical element, and $S_L$ ($\lambda$) is a sensitivity function of L cones obtained by converting a sensitivity function of L cones described in CIE S/026E: 2018 such that a maximum sensitivity thereof is identical to a maximum sensitivity of an x bar ($\lambda$) in CIE 1931 color-matching functions.

<3> An optical element, comprising a functional layer, wherein:

the functional layer has, in a spectrum measured in accordance with CIE 1976, (A) a local maximum absorption wavelength a that is present within a range of from 450 nm to 540 nm, and (B) a local maximum absorption wavelength b that is present within a range of from more than 540 nm to 630 nm,
a transmittance at the local maximum absorption wavelength a is from 2% to 50%,
a transmittance at the local maximum absorption wavelength b is from 10% to 90%, and
a sum of the transmittance at the local maximum absorption wavelength a and the transmittance at the local maximum absorption wavelength b is from 20% to 120%.

<4> The optical element according to <3>, wherein a value obtained by subtracting the local maximum absorption wavelength a from the local maximum absorption wavelength b is from 50 nm to 110 nm.
<5> The optical element according to <3> or <4>, wherein the functional layer contains at least one polymer selected from the group consisting of polyurethanes, polythiourethanes, polysulfides, polycarbonates, poly(meth)acrylates, and poly(thio)epoxides.
<6> An eyeglass lens, comprising the optical element according to any one of <1> to <5>.
<7> The eyeglass lens according to <6>, having a luminous transmittance of from 8% to 90%.
<8> An autonomic nerve regulation method, comprising regulating autonomic nerves through an optical element, in which Stim (ipRGC) represented by the following Formula 1 is from 10.0 to 68.0:

$$\text{Stim}(\text{ipRGC}) = \frac{\int_{390nm}^{780nm} \{RI_{D65}(\lambda) \cdot \tau(\lambda) \cdot S_{mel}(\lambda)\} d\lambda}{\int_{390nm}^{780nm} \{RI_{D65}(\lambda) \cdot S_{mel}(\lambda)\} d\lambda} \quad \text{Formula 1}$$

wherein, in Formula 1, $RI_{D65}$ ($\lambda$) is a spectral irradiance of illuminant D65, $\tau$ ($\lambda$) is a spectral transmittance of the optical element, and $S_{mel}$ ($\lambda$) is a sensitivity function of ipRGCs obtained by converting a sensitivity function of ipRGCs described in CIE S/026E: 2018 such that a maximum sensitivity thereof is identical to a maximum sensitivity of a z bar ($\lambda$) in CIE 1931 color-matching functions.
<9> The autonomic nerve regulation method according to <8>, wherein, in the optical element, a sum of the Stim (ipRGC) and Stim (L) represented by the following Formula 2 is from 20.0 to 135.0:

$$\text{Stim}(L) = \frac{\int_{390nm}^{780nm} \{RI_{D65}(\lambda) \cdot \tau(\lambda) \cdot S_L(\lambda)\} d\lambda}{\int_{390nm}^{780nm} \{RI_{D65}(\lambda) \cdot S_L(\lambda)\} d\lambda} \quad \text{Formula 2}$$

wherein, in Formula 2, $RI_{D65}$ ($\lambda$) is the spectral irradiance of illuminant D65, $\tau$ ($\lambda$) is the spectral transmittance of the optical element, and $S_L$ ($\lambda$) is a sensitivity function of L cones obtained by converting a sensitivity function of L cones described in CIE S/026E: 2018 such that a maximum sensitivity thereof is identical to a maximum sensitivity of an x bar ($\lambda$) in CIE 1931 color-matching functions.
<10> The autonomic nerve regulation method according to <8> or <9>, comprising regulating at least one selected

from the group consisting of a heart rate, a ratio (LF/HF) of a low-frequency component LF to a high-frequency component HF of the heart rate, and pNN 50.

<11> An evaluation method for an optical element for regulating autonomic nerves, the method comprising:

a preparation step of preparing an optical element, in which Stim (ipRGC) represented by the following Formula 1 is from 10.0 to 68.0; and

an evaluation step of carrying out evaluation by measuring a heart rate variability of a subject before and after irradiating the subject with artificial sunlight through the optical element for a predetermined time in a dark place with illuminance of 5 lux or less:

$$\mathrm{Stim(ipRGC)} = \frac{\int_{390nm}^{780nm} \{RI_{D65}(\lambda) \cdot \tau(\lambda) \cdot S_{mel}(\lambda)\} d\lambda}{\int_{390nm}^{780nm} \{RI_{D65}(\lambda) \cdot S_{mel}(\lambda)\} d\lambda} \qquad \text{Formula 1}$$

wherein, in Formula 1, $RI_{D65}$ (λ) is a spectral irradiance of illuminant D65, τ (λ) is a spectral transmittance of the optical element, and $S_{mel}$ (λ) is a sensitivity function of ipRGCs obtained by converting a sensitivity function of ipRGCs described in CIE S/026E: 2018 such that a maximum sensitivity thereof is identical to a maximum sensitivity of a z bar (λ) in CIE 1931 color-matching functions.

<12> The evaluation method for an optical element according to <11>, wherein the evaluation step includes regulating autonomic nerves by comparing a heart rate variability of the subject in the dark place with illuminance of 5 lux or less in a state of not using the optical element and not irradiating the subject with the artificial sunlight, with the heart rate variability of the subject before and after irradiating the subject with the artificial sunlight through the optical element for the predetermined time in the dark place with illuminance of 5 lux or less.

Advantageous Effects of Invention

According to an embodiment of the disclosure, an optical element capable of regulating autonomic nerves, an eyeglass lens, an autonomic nerve regulation method, and an evaluation method for an optical element can be provided.

BRIEF DESCRIPTION OF DRAWINGS

Fig. 1 is a graph showing spectra measured in accordance with CIE 1976 for eyeglass lenses of Examples 1 to 3.

Fig. 2 is a graph showing spectra measured in accordance with CIE 1976 for eyeglass lenses of Example 4, Example 5, Comparative Example 1, and Comparative Example 2.

DESCRIPTION OF EMBODIMENTS

In the disclosure, a numeral value range represented by "(a value) to (a value)" means a range including numeral values described before and after "to" as a lower limit value and an upper limit value.

In the disclosure, in a case in which there are a plurality of substances corresponding to each component in a composition, the amount of each component in the composition means the total amount of the plurality of substances present in the composition unless otherwise particularly specified.

In the disclosure, "(meth)acryloyl" means acryloyl or methacryloyl, and "(meth)acrylate" means acrylate or methacrylate.

In numerical ranges described in stages, in the disclosure, the upper limit value or the lower limit value described in one numerical range may be replaced with the upper limit value or the lower limit value of another numerical range described in stages. The upper limit value or lower limit value of the numerical range described in the disclosure may be replaced with values shown in the Examples.

The term "step" in the disclosure includes not only a separate step but also a step which cannot be clearly distinguished from other steps as long as the desired object of the step is achieved.

<Optical element>

Examples of an optical element of the disclosure include the following embodiment A and embodiment B.

Hereinafter, the embodiment A and the embodiment B will be described.

In the disclosure, autonomic nerves include two nervous systems of a sympathetic nerve and a parasympathetic nerve. Sympathetic and parasympathetic nerve activities are balanced and affect the activities of organs such as circulatory, digestive, and respiratory organs.

The regulation of the autonomic nerves means, for example, maintaining the homeostasis of the activity of each organ in a scene where autonomic nerve activity is disturbed, specifically, suppressing a rapid increase or decrease in a heart rate, a rapid increase or decrease in the ratio of a low-frequency component LF to a high-frequency component HF (LF/HF) of the heart rate, and a rapid increase or decrease in pNN 50.

The scene where the autonomic nerves are disturbed is, for example, a scene where sudden light stimulation is given. Specifically, examples of the scene include a scene where a person leaves the room to the outside and a scene where a headlight of a vehicle enters the eyes at night.

<Embodiment A>

In the optical element of the embodiment A, Stim (ipRGC) represented by the following Formula 1 is from 10.0 to 68.0.

$$\mathrm{Stim(ipRGC)} = \frac{\int_{390nm}^{780nm}\left\{RI_{D65}(\lambda)\ \cdot \tau(\lambda)\ \cdot S_{mel}(\lambda)\right\}d\lambda}{\int_{390nm}^{780nm}\left\{RI_{D65}(\lambda)\ \cdot S_{mel}(\lambda)\right\}d\lambda} \qquad \text{Formula 1}$$

In Formula 1, $RI_{D65}$ (λ) is a spectral irradiance of illuminant D65, τ (λ) is a spectral transmittance of an optical element, and $S_{mel}$ (λ) is a sensitivity function of ipRGCs obtained by converting a sensitivity function of ipRGCs described in CIE S/026E: 2018 such that a maximum sensitivity thereof is identical to a maximum sensitivity of a z bar (λ) in CIE 1931 color-matching functions.

The z bar (λ) in CIE 1931 color-matching functions means the function of a z component in CIE 1931 color-matching functions.

Note that τ (λ) is the spectral transmittance of the optical element, and the unit is %.

(Stim(ipRGC))

In the optical element of the embodiment A, Stim (ipRGC) represented by the above Formula 1 is from 10.0 to 68.0.

Stim (ipRGC) represents a stimulation amount for ipRGCs.

When Stim (ipRGC) represented by Formula 1 is 10.0 or more, visibility is obtained.

From the above viewpoint, Stim (ipRGC) represented by Formula 1 is preferably 15.0 or more, more preferably 20.0 or more, and still more preferably 30.0 or more.

When Stim (ipRGC) represented by Formula 1 is 68.0 or less, an increase in a heart rate due to light stimulation can be suppressed.

From the above viewpoint, Stim (ipRGC) represented by Formula 1 is preferably 65.0 or less, and more preferably 60.0 or less.

In the optical element in the embodiment A, Stim (L) represented by the following Formula 2 is preferably from 40.0 to 85.0.

Stim (L) represents a stimulation amount for L cones.

$$\mathrm{Stim}(L) = \frac{\int_{390nm}^{780nm}\left\{RI_{D65}(\lambda)\ \cdot \tau(\lambda)\ \cdot S_{L}(\lambda)\right\}d\lambda}{\int_{390nm}^{780nm}\left\{RI_{D65}(\lambda)\ \cdot S_{L}(\lambda)\right\}d\lambda} \qquad \text{Formula 2}$$

In Formula 2, $RI_{D65}$ (λ) is a spectral irradiance of illuminant D65, τ (λ) is a spectral transmittance of an optical element, and $S_L$ (λ) is a sensitivity function of L cones obtained by converting the sensitivity function of L cones described

in CIE S/026E: 2018 such that a maximum sensitivity thereof is identical to a maximum sensitivity of the x bar (λ) in CIE 1931 color-matching functions.

The x bar (λ) in CIE 1931 color-matching functions means the function of an x component in CIE 1931 color-matching functions.

Note that τ (λ) is the spectral transmittance of the optical element, and the unit is %.

When Stim (L) represented by Formula 2 is 40.0 or more, visibility is obtained.

From the above viewpoint, Stim (L) represented by Formula 2 is preferably 50.0 or more, and more preferably 55.0 or more.

When Stim (L) represented by Formula 2 is 85.0 or less, an increase in a heart rate due to light stimulation can be suppressed.

From the above viewpoint, Stim (L) represented by Formula 2 is preferably 80.0 or less, and more preferably 77.0 or less.

In the optical element of the embodiment A, the sum of Stim (ipRGC) and Stim (L) represented by the above Formula 2 is preferably from 20.0 to 135.0.

When the sum of Stim (ipRGC) and Stim (L) represented by Formula 2 is 20.0 or more, visibility is obtained.

From the above viewpoint, the sum of Stim (ipRGC) and Stim (L) represented by Formula 2 is preferably 20.0 or more, more preferably 40.0 or more, still more preferably 60.0 or more, and particularly preferably 80.0 or more.

When the sum of Stim (ipRGC) and Stim (L) represented by Formula 2 is 135.0 or less, an increase in a heart rate due to light stimulation can be suppressed.

From the above viewpoint, the sum of Stim (ipRGC) and Stim (L) represented by Formula 2 is preferably 130.0 or less, more preferably 128.0 or less, and still more preferably 125.0 or less.

<Embodiment B>

The optical element of the embodiment B includes a functional layer, wherein the functional layer has, in a spectrum measured in accordance with CIE 1976, (A) a local maximum absorption wavelength a present within a range of from 450 nm to 540 nm, and (B) a local maximum absorption wavelength b present within a range of from more than 540 nm to 630 nm, a transmittance at the local maximum absorption wavelength a is from 2% to 50%, a transmittance at the local maximum absorption wavelength b is from 10% to 90%, and a sum of the transmittance (%) at the local maximum absorption wavelength a and the transmittance (%) at the local maximum absorption wavelength b is from 20% to 120%.

The present inventors have conducted various studies and considerations regarding the control of light incident on a photoreceptor, and have conducted intensive studies. As a result, the inventors have found that there is a possibility that the autonomic nerves can be affected by controlling the light incident on the photoreceptor. In particular, the inventors have found that a heart rate, HF/LF, and pNN 50 are affected.

As a result of further research based on the above findings, the inventors have found that an optical element (for example, eyeglass lens) in which Stim (ipRGC) represented by the following Formula 1 is within a certain range can regulate the autonomic nerves.

$$\mathrm{Stim(ipRGC)} = \frac{\int_{390nm}^{780nm} \{RI_{D65}(\lambda) \cdot \tau(\lambda) \cdot S_{mel}(\lambda)\} d\lambda}{\int_{390nm}^{780nm} \{RI_{D65}(\lambda) \cdot S_{mel}(\lambda)\} d\lambda} \qquad \text{Formula 1}$$

In Formula 1, $RI_{D65}$ (λ) is a spectral irradiance of illuminant D65, τ (λ) is a spectral transmittance of an optical element, and $S_{mel}$ (λ) is a sensitivity function of ipRGCs obtained by converting a sensitivity function of ipRGCs described in CIE S/026E: 2018 such that a maximum sensitivity thereof is identical to a maximum sensitivity of a z bar (λ) in CIE 1931 color-matching functions.

Note that τ (λ) is the spectral transmittance of the optical element, and the unit is %.

As a result of pursuing such an optical element, the optical element of the embodiment B has been completed.

The optical element of the embodiment B includes the above configuration, and thereby Stim (ipRGC) represented by Formula 1 can be preferably regulated to fall within a range of from 10.0 to 68.0.

(Functional layer)

The optical element of the embodiment B includes a functional layer, wherein the functional layer has, in a spectrum measured in accordance with CIE 1976, (A) a local maximum absorption wavelength a present within a range

of from 450 nm to 540 nm, and (B) a local maximum absorption wavelength b present within a range of from more than 540 nm to 630 nm, a transmittance at the local maximum absorption wavelength a is from 2% to 50%, a transmittance at the local maximum absorption wavelength b is from 10% to 90%, and a sum of the transmittance at the local maximum absorption wavelength a and the transmittance at the local maximum absorption wavelength b is from 20% to 120%.

In the optical element of the embodiment B, only one local maximum absorption wavelength a may be present within the range of from 450 nm to 540 nm, or two or more local maximum absorption wavelengths a may be present.

In the optical element of the embodiment B, only one local maximum absorption wavelength b may be present within the range of from more than 540 nm to 630 nm, or two or more local maximum absorption wavelengths b may be present.

The functional layer has (A) a local maximum absorption wavelength a present within a range of from 450 nm to 540 nm in a spectrum measured in accordance with CIE 1976.

This makes it easy to regulate Stim (ipRGC) to fall within a range of from 10.0 to 68.0.

From the above viewpoint, the local maximum absorption wavelength a is preferably present within a range of from 450 nm to 530 nm, and more preferably within a range of from 460 nm to 530 nm.

The transmittance at the local maximum absorption wavelength a is from 2% to 50%, and preferably from 10% to 50%.

When the transmittance at the local maximum absorption wavelength a is 2% or more, visibility can be favorably maintained.

From the above viewpoint, the transmittance at the local maximum absorption wavelength a is preferably 20% or more.

When the transmittance at the local maximum absorption wavelength a is 50% or less, an increase in a heart rate due to light stimulation can be suppressed.

From the above viewpoint, the transmittance at the local maximum absorption wavelength a is preferably 45% or less, and more preferably 40% or less.

The functional layer has (B) a local maximum absorption wavelength b present within a range of from more than 540 nm to 630 nm in a spectrum measured in accordance with CIE 1976.

This makes it easy to regulate the sum of Stim (ipRGC) and Stim (L) to fall within a range of from 20.0 to 135.0.

From the above viewpoint, the local maximum absorption wavelength b is preferably present within a range of from 540 nm to 620 nm, and more preferably within a range of from 540 nm to 610 nm.

The transmittance at the local maximum absorption wavelength b is from 10% to 90%.

When the transmittance at the local maximum absorption wavelength b is 10% or more, visibility can be favorably maintained.

From the above viewpoint, the transmittance at the local maximum absorption wavelength b is preferably 15% or more, more preferably 20% or more, and still more preferably 30% or more.

When the transmittance at the local maximum absorption wavelength b is 90% or less, an increase in a heart rate due to light stimulation can be suppressed.

From the above viewpoint, the transmittance at the local maximum absorption wavelength b is preferably 80% or less, and more preferably 70% or less.

In the optical element of the embodiment B, the sum of the transmittance at the local maximum absorption wavelength a and the transmittance at the local maximum absorption wavelength b is from 20% to 120%.

When the sum of the transmittance at the local maximum absorption wavelength a and the transmittance at the local maximum absorption wavelength b is 20% or more, visibility can be favorably maintained.

From the above viewpoint, the sum of the transmittance at the local maximum absorption wavelength a and the transmittance at the local maximum absorption wavelength b is preferably 30% or more, more preferably 40% or more, and still more preferably 50% or more.

When the sum of the transmittance at the local maximum absorption wavelength a and the transmittance at the local maximum absorption wavelength b is 120% or less, an amount of stimulation to ipRGCs and L cones is reduced, and an increase in a heart rate due to light stimulation can be suppressed.

From the above viewpoint, the sum of the transmittance at the local maximum absorption wavelength a and the transmittance at the local maximum absorption wavelength b is preferably 115% or less, and more preferably 110% or less.

In the functional layer, a value obtained by subtracting the local maximum absorption wavelength a from the local maximum absorption wavelength b is preferably from 50 nm to 110 nm, and more preferably from 55 nm to 107 nm.

The thickness of the functional layer in the optical element of the embodiment B is not particularly limited, and may be, for example, from 0.5 μm to 10 mm, from 0.5 mm to 10 mm, from 1 mm to 5 mm, or from 1.5 mm to 3 mm. As an example, the thickness of the functional layer in the embodiment B may be 2 mm.

The thickness of the functional layer in the optical element of the embodiment B means the maximum thickness.

In the optical element of the embodiment B, for example, the types, amounts, and combination and the like of

colorants contained in the optical element, the types, amounts, and combination and the like of additives such as an ultraviolet absorber contained in the optical element if necessary, and the types, amounts, and combination and the like of polymers contained in the optical element if necessary may be regulated if appropriate to regulate Stim (ipRGC) represented by Formula 1 to fall within a range of from 10.0 to 68.0.

(First colorant)

The optical element of the embodiment B preferably contains a first colorant having a local maximum absorption wavelength positioned within a range of from 450 nm to 540 nm. The first colorant may be used singly or in combination of two or more kinds thereof.

The first colorant preferably contains at least one of a porphyrin-based compound or a merocyanine-based compound, and more preferably contains a porphyrin-based compound.

When the first colorant contains the porphyrin-based compound or the merocyanine-based compound or the like, the first colorant suitably absorbs light having a wavelength within a range of from 450 nm to 540 nm.

The porphyrin-based compound preferably contains a compound represented by the following General Formula (2).

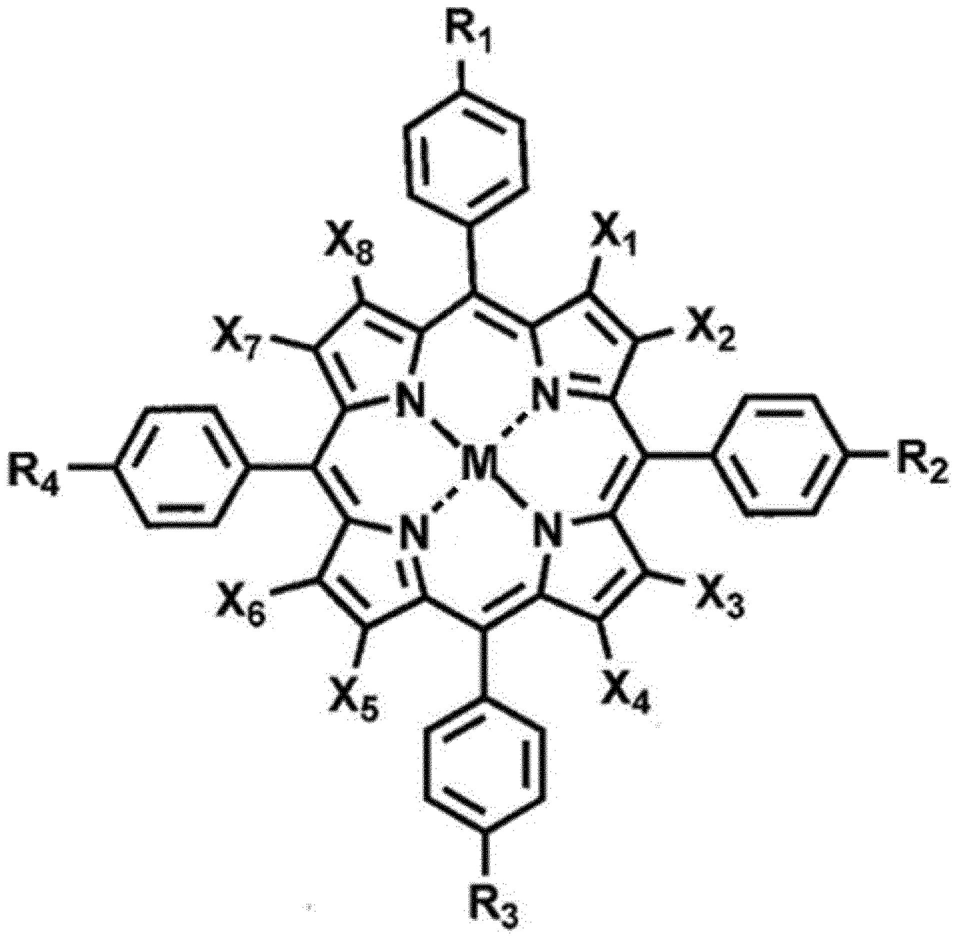

(2)

In General Formula (2), $X_1$ to $X_8$ each independently represent a hydrogen atom, a halogen atom, a nitro group, a cyano group, a hydroxy group, an amino group, a carboxyl group, a sulfonic acid group, a linear, branched or cyclic alkyl group having 1 to 20 carbon atoms, a linear, branched or cyclic alkenyl group having 2 to 20 carbon atoms, a linear, branched or cyclic alkynyl group having 2 to 20 carbon atoms, an alkoxy group having 1 to 20 carbon atoms, an aryloxy group having 6 to 20 carbon atoms, a monoalkylamino group having 1 to 20 carbon atoms, a dialkylamino group having 2 to 20 carbon atoms, an aralkyl group having 7 to 20 carbon atoms, an aryl group having 6 to 20 carbon atoms, a heteroaryl group having 6 to 20 carbon atoms, an alkylthio group having 6 to 20 carbon atoms, or an arylthio group having 6 to 20 carbon atoms. Ri to $R_4$ each independently represent a hydrogen atom, or a linear or branched alkyl group, and M represents two hydrogen atoms, a divalent metal atom, a trivalent substituted metal atom, a tetravalent substituted metal atom, a hydroxide metal atom, or an oxide metal atom.

At least one of $X_1$ to $X_8$ is preferably other than a hydrogen atom, and is preferably a halogen atom, a linear, branched or cyclic alkyl group having 1 to 20 carbon atoms, a linear, branched or cyclic alkenyl group having 2 to 20 carbon atoms, or a linear, branched or cyclic alkynyl group having 2 to 20 carbon atoms.

Examples of the halogen atom in $X_1$ to $X_8$ include a fluorine atom, a chlorine atom, a bromine atom, and an iodine atom, and a fluorine atom, a chlorine atom, or a bromine atom is preferable, and a fluorine atom or a bromine atom is more preferable.

Ri to $R_4$ are each independently preferably a hydrogen atom, or a linear or branched alkyl group having 1 to 8 carbon atoms.

M is preferably Cu, Zn, Fe, Co, Ni, Pt, Pd, Mn, Mg, Mn(OH), $Mn(OH)_2$, VO, or TiO, and more preferably Ni, Pd, or VO.

Preferred configurations of the linear, branched or cyclic alkyl group having 1 to 20 carbon atoms, the linear, branched or cyclic alkenyl group having 2 to 20 carbon atoms, and the linear, branched or cyclic alkynyl group having 2 to 20 carbon atoms in $X_1$ to $X_8$ are the same as those of $A_1$ to As in General Formula (1) described above.

Examples of the linear or branched alkyl group having 1 to 8 carbon atoms in Ri to $R_4$ include a methyl group, an ethyl group, a n-propyl group, an isopropyl group, a n-butyl group, an isobutyl group, a tert-butyl group, a n-pentyl group, an isopentyl group, a neopentyl group, a tert-pentyl group, a 1,2-dimethylpropyl group, a 1-methylbutyl group, a 2-methylbutyl group, a n-hexyl group, a 2-methylpentyl group, a 4-methylpentyl group, a 4-methyl-2-pentyl group, a 1,2-dimethylbutyl group, a 2,3-dimethylbutyl group, a 2-ethylbutyl group, a n-heptyl group, a 3-methylhexyl group, a 5-methylhexyl group, a 2,4-dimethylpentyl group, a n-octyl group, a tert-octyl group, a 2-ethylhexyl group, a 2-propylpentyl group, and a 2,5-dimethylhexyl group.

Among them, a methyl group, an ethyl group, a n-propyl group, an isopropyl group, a n-butyl group, an isobutyl group, a tert-butyl group, a n-pentyl group, an isopentyl group, a neopentyl group, a 1,2-dimethylpropyl group, a 1-methylbutyl group, a n-hexyl group, a 1,2-dimethylbutyl group, a 2-ethylbutyl group, a n-heptyl group, a n-octyl group, or a 2-ethylhexyl group is preferable, and a methyl group, an ethyl group, a n-propyl group, an isopropyl group, a n-butyl group, an isobutyl group, a tert-butyl group, a n-pentyl group, an isopentyl group, a n-hexyl group, a 1,2-dimethylbutyl group, a 2-ethylbutyl group, a n-heptyl group, or a n-octyl group is more preferable.

The porphyrin-based compound that can be used for the optical element of the embodiment B can be produced with reference to a method known per se. For example, it can be produced by the method described in Octabromotetraphenylporphyrin and Its Metal Derivatives (Inorg. Chem. 1991, 30, 239-245).

The compound represented by General Formula (2) can be produced, for example, by using a pyrrole compound and an aldehyde compound, obtaining a compound synthesized by a Rothemund reaction through a dehydration condensation reaction using an acid catalyst and an oxidation reaction using an oxidizing agent, and then reacting the compound with a metal or a metal salt (for example, an acetylacetone complex, an acetate of a metal) in a solvent. Examples of the pyrrole compound include compounds represented by General Formulas (B-1) to (B-4), and examples of the aldehyde compound include compounds represented by General Formulas (C-1) to (C-4). Examples of the acid catalyst include propionic acid, a boron trifluoride/ethyl ether complex, and trifluoroacetic acid, and examples of the oxidizing agent include 2,3-dichloro-5,6-dicyano-1,4-benzoquinone.

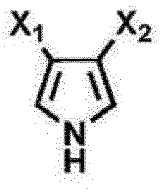
(B-1)

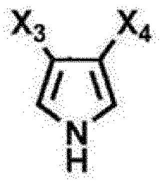
(B-2)

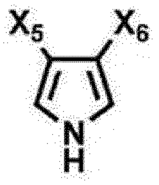
(B-3)

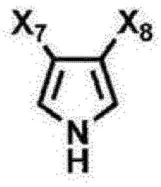
(B-4)

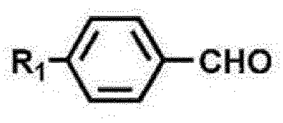
(C-1)

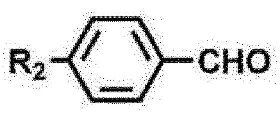
(C-2)

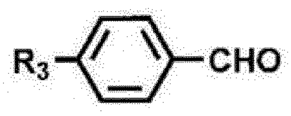
(C-3)

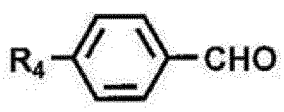
(C-4)

$X_1$ to $X_8$ and $R_1$ to $R_4$ in General Formulae (B-1) to (B-4) and General Formulae (C-1) to (C-4) are the same as $X_1$ to $X_8$ and Ri to $R_4$ in General Formula (2).

The compound represented by General Formula (2) may be used singly or in a mixture containing two or more

kinds of isomers.

In a case where the optical element of the embodiment B contains a porphyrin-based compound, the content of the porphyrin-based compound is preferably from 1.5 ppm to 60 ppm, and more preferably from 5 ppm to 50 ppm.

In a case where the optical element of the embodiment B contains a merocyanine-based compound, the content of the merocyanine-based compound is preferably from 1.5 ppm to 14 ppm, and more preferably from 2 ppm to 12 ppm.

As the first colorant, for example, UVY-0026 (manufactured by Yamamoto Chemicals, Inc.), UVY-1023 (manufactured by Yamamoto Chemicals, Inc.), FDB-003 (manufactured by Yamada Chemical Co., Ltd.), and ABS 430 (manufactured by Luxottica) and the like can be used as commercially available products.

(Second colorant)

The optical element of the embodiment B preferably contains a second colorant having a local maximum absorption wavelength positioned within a range of from more than 540 nm to 630 nm. The second colorant may be used singly or in combination of two or more kinds thereof.

The second colorant preferably contains a tetraazaporphyrin-based metal complex compound.

The tetraazaporphyrin-based metal complex compound is not particularly limited as long as it is a metal complex compound having a tetraazaporphyrin skeleton and a metal atom, and is preferably, for example, a compound represented by the following General Formula (1).

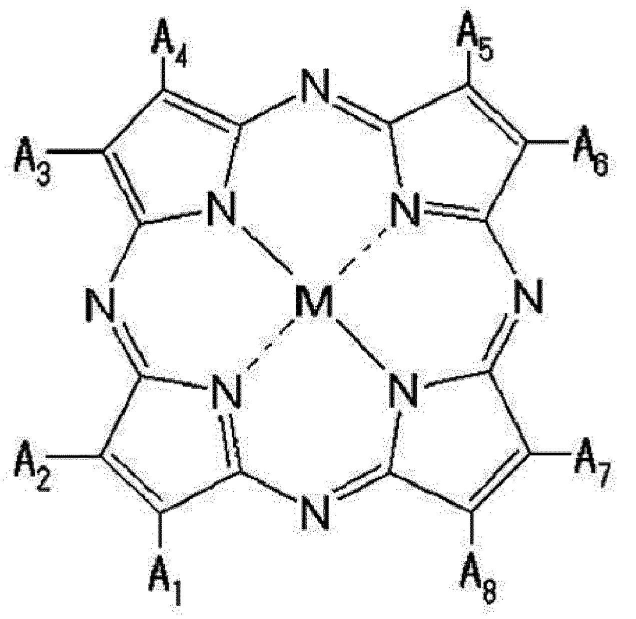

(1)

In General Formula (1), $A_1$ to $A_8$ each independently represent a hydrogen atom, a halogen atom, a nitro group, a cyano group, a hydroxy group, an amino group, a carboxyl group, a sulfonic acid group, a linear, branched or cyclic alkyl group having 1 to 20 carbon atoms, a linear, branched or cyclic alkenyl group having 2 to 20 carbon atoms, a linear, branched or cyclic alkynyl group having 2 to 20 carbon atoms, an alkoxy group having 1 to 20 carbon atoms, an aryloxy group having 6 to 20 carbon atoms, a monoalkylamino group having 1 to 20 carbon atoms, a dialkylamino group having 2 to 20 carbon atoms, an aralkyl group having 7 to 20 carbon atoms, an aryl group having 6 to 20 carbon atoms, a heteroaryl group having 6 to 20 carbon atoms, an alkylthio group having 6 to 20 carbon atoms, or an arylthio group having 6 to 20 carbon atoms. $A_1$ and $A_2$, $A_3$ and $A_4$, As and $A_6$, and $A_7$ and $A_8$ may each independently form a ring excluding an aromatic ring. M represents a divalent metal atom, a trivalent substituted metal atom, a tetravalent substituted metal atom, a hydroxide metal atom, or an oxide metal atom.

Examples of the halogen atom in $A_1$ to $A_8$ include a fluorine atom, a chlorine atom, a bromine atom, and an iodine atom, and a fluorine atom, a chlorine atom, or a bromine atom is preferable, and a fluorine atom or a bromine atom is more preferable.

Examples of the linear, branched or cyclic alkyl group having 1 to 20 carbon atoms in $A_1$ to $A_8$ include a methyl group, an ethyl group, a n-propyl group, an isopropyl group, a n-butyl group, an isobutyl group, a tert-butyl group, a n-pentyl group, an isopentyl group, a neopentyl group, a tert-pentyl group, a 1,2-dimethylpropyl group, a 1-methylbutyl group, a 2-methylbutyl group, a n-hexyl group, a 2-methylpentyl group, a 4-methylpentyl group, a 4-methyl-2-pentyl group, a 1,2-dimethylbutyl group, a 2,3-dimethylbutyl group, a 2-ethylbutyl group, a n-heptyl group, a 3-methylhexyl

group, a 5-methylhexyl group, a 2,4-dimethylpentyl group, a n-octyl group, a tert-octyl group, a 2-ethylhexyl group, a 2-propylpentyl group, a 2,5-dimethylhexyl group, a cyclopentyl group, a cyclohexyl group, a cycloheptyl group, a cyclooctyl group, a norbornyl group, and an isobornyl group.

Examples of the linear, branched or cyclic alkenyl group in $A_1$ to $A_8$ include a vinyl group, a 1-methylvinyl group, a propenyl group, a 2-butenyl group, and a 2-pentenyl group.

Examples of the linear, branched or cyclic alkynyl group in $A_1$ to $A_8$ include an ethynyl group, a propynyl group, a butynyl group, a 1,3-butadiynyl group, a 2-pentynyl group, a 2,4-pentadiynyl group, a 2-hexynyl group, a 3,3-dimethyl-1-butynyl group, a 3-heptynyl group, and a 4-octynyl group.

In General Formula (1), M is preferably a divalent metal atom, and more preferably divalent copper. Specific examples of the tetraazaporphyrin-based metal complex compound include a tetra-t-butyl-tetraazaporphyrin-copper complex represented by the following Formula (1a).

As the second colorant, for example, PD-311S (manufactured by Yamamoto Chemicals, Inc.), FDG-002 (manufactured by Yamada Chemical Co., Ltd.), and ABS 594 (manufactured by Luxottica) and the like can be used as commercially available products.

In a case where the optical element of the embodiment B contains the second colorant, the content of the second colorant is preferably from 5 ppm to 18 ppm, and more preferably from 8 ppm to 15 ppm.

(Polymer)

The functional layer in the embodiment B preferably contains a polymer.

In the embodiment B, the polymer to be used may be a commercially available product, or a monomer or a polymer obtained from the monomer or the like may be used.

In the embodiment B, the polymer can be used without particular limitation, and is preferably a transparent polymer.

Hereinafter, the polymer, and the monomer for obtaining the polymer will be described.

The polymer is not particularly limited, and examples thereof include polyurethanes, polythiourethanes, polysulfides, polycarbonates, poly(meth)acrylates, polyolefins, cyclic polyolefins, polyallyls, polyurethane ureas, polyene-polythiol polymers, ring-opened metathesis polymers, polyesters, and epoxy resins.

The polymer may be used singly or in combination of two or more kinds thereof.

The functional layer preferably contains at least one polymer selected from the group consisting of polyurethanes, polythiourethanes, polysulfides, polycarbonates, poly(meth)acrylates, and poly(thio)epoxides, and more preferably contains a polythiourethane. These polymers are materials having high transparency, and can be suitably used for eyeglass lens applications.

The polyurethane contains a constituent unit derived from a polyisocyanate compound and a constituent unit derived from a polyol compound. The polythiourethane contains a constituent unit derived from a polyisocyanate compound and a constituent unit derived from a polythiol compound.

Examples of the polyisocyanate compound include aliphatic polyisocyanate compounds such as 1,6-hexamethylene diisocyanate, 1,5-pentamethylene diisocyanate, 2,2,4-trimethylhexane diisocyanate, 2,4,4-trimethylhexamethylene diisocyanate, lysine diisocyanatomethyl ester, lysine triisocyanate, m-xylylene diisocyanate, $\alpha,\alpha,\alpha',\alpha'$-tetramethylxylylene diisocyanate, bis(isocyanatomethyl)naphthaline, mesitylene triisocyanate, bis(isocyanatomethyl)sulfide, bis(isocyanatoethyl)sulfide, bis(isocyanatomethyl)disulfide, bis(isocyanatoethyl)disulfide, bis(isocyanatomethylthio)methane, bis(isocyanatoethylthio)methane, bis(isocyanatoethylthio)ethane, and bis(isocyanatomethylthio)ethane; alicyclic polyisocyanate compounds such as isophorone diisocyanate, 1,3-bis(isocyanatomethyl)cyclohexane, 1,4-bis(isocyanatomethyl)cyclohexane, dicyclohexylmethane diisocyanate, cyclohexane diisocyanate, methylcyclohexane diisocyanate, dicyclohexyldimethylmethane isocyanate, 2,5-bis(isocyanatomethyl)bicyclo-[2.2.1]-heptane, 2,6-bis(isocyanatomethyl)bicyclo-[2.2.1]-heptane, 3,8-bis(isocyanatomethyl)tricyclodecane, 3,9-bis(isocyanatomethyl)tricyclodecane, 4,8-bis(isocyanatomethyl)tricyclodecane, and 4,9-bis(isocyanatomethyl)tricyclodecane; aromatic polyisocyanate compounds such as naphthalene diisocyanate, m-phenylene diisocyanate, p-phenylene diisocyanate, 2,4-tolylene diisocyanate, 2,6-tolylene diisocyanate, biphenyl diisocyanate, diphenylmethane-2,2'-diisocyanate, diphenylmethane-2,4'-diisocyanate, diphenylmethane-4,4'-diisocyanate, benzene triisocyanate, and diphenylsulfide-4,4-diisocyanate; and heterocyclic polyisocyanate compounds such as 2,5-diisocyanatothiophene, 2,5-bis(isocyanatomethyl)thiophene, 2,5-diisocyanatotetrahydrothiophene, 2,5-bis(isocyanatomethyl)tetrahydrothiophene, 3,4-bis(isocyanatomethyl)tetrahydrothiophene, 2,5-diisocyanato-1,4-dithiane, 2,5-bis(isocyanatomethyl)-1,4-dithiane, 4,5-diisocyanato-1,3-dithiolane, and 4,5-bis(isocyanatomethyl)-1,3-dithiolane, and at least one selected from these can be used.

The polyol compound is one or more aliphatic or alicyclic alcohols. Specific examples thereof include linear or branched aliphatic alcohols, alicyclic alcohols, and alcohols obtained by adding ethylene oxide, propylene oxide, or $\varepsilon$-caprolactone to these alcohols, and at least one selected from these alcohols can be used.

Examples of the linear or branched aliphatic alcohol include ethylene glycol, diethylene glycol, triethylene glycol,

propylene glycol, dipropylene glycol, tripropylene glycol, 1,3-propanediol, 2,2-dimethyl-1,3-propanediol, 2,2-diethyl-1,3-propanediol, 1,2-butanediol, 1,3-butanediol, 1,4-butanediol, 3-methyl-1,3-butanediol, 1,2-pentanediol, 1,3-pentanediol, 1,5-pentanediol, 2,4-pentanediol, 2-methyl-2,4-pentanediol, 3-methyl-1,5-pentanediol, 1,6-hexanediol, 2,5-hexanediol, glycerol, diglycerol, polyglycerol, trimethylolpropane, pentaerythritol, and di(trimethylolpropane).

Examples of the alicyclic alcohol include 1,2-cyclopentanediol, 1,3-cyclopentanediol, 3-methyl-1,2-cyclopentanediol, 1,2-cyclohexanediol, 1,3-cyclohexanediol, 1,4-cyclohexanediol, 4,4'-bicyclohexanol, and 1,4-cyclohexanedimethanol, and at least one selected from these can be used.

Compounds obtained by adding ethylene oxide, propylene oxide, or ε-caprolactone to these alcohols may be used. Examples thereof include an ethylene oxide adduct of glycerol, an ethylene oxide adduct of trimethylolpropane, an ethylene oxide adduct of pentaerythritol, a propylene oxide adduct of glycerol, a propylene oxide adduct of trimethylolpropane, a propylene oxide adduct of pentaerythritol, caprolactone-modified glycerol, caprolactone-modified trimethylolpropane, and caprolactone-modified pentaerythritol, and at least one selected from these can be used.

Examples of the polythiol compound include aliphatic polythiol compounds such as methane dithiol, 1,2-ethanedithiol, 1,2,3-propanetrithiol, 1,2-cyclohexanedithiol, bis(2-mercaptoethyl)ether, tetrakis(mercaptomethyl)methane, diethylene glycol bis(2-mercaptoacetate), diethylene glycol bis(3-mercaptopropionate), ethylene glycol bis(2-mercaptoacetate), ethylene glycol bis(3-mercaptopropionate), trimethylolpropane tris(2-mercaptoacetate), trimethylolpropane tris(3-mercaptopropionate), trimethylolethane tris(2-mercaptoacetate), trimethylolethane tris(3-mercaptopropionate), pentaerythritol tetrakis(2-mercaptoacetate), pentaerythritol tetrakis(3-mercaptopropionate), bis(mercaptomethyl)sulfide, bis(mercaptomethyl)disulfide, bis(mercaptoethyl)sulfide, bis(mercaptoethyl)disulfide, bis(mercaptopropyl)sulfide, bis(mercaptomethylthio)methane, bis(2-mercaptoethylthio)methane, bis(3-mercaptopropylthio)methane, 1,2-bis(mercaptomethylthio)ethane, 1,2-bis(2-mercaptoethylthio)ethane, 1,2-bis(3-mercaptopropylthio)ethane, 1,2,3-tris(mercaptomethylthio)propane, 1,2,3-tris(2-mercaptoethylthio)propane, 1,2,3-tris(3-mercaptopropylthio)propane, 4-mercaptomethyl-1,8-dimercapto-3,6-dithiaoctane, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, tetrakis(mercaptmethylthiomethyl)methane, tetrakis(2-mercaptoethylthiomethyl)methane, tetrakis(3-mercaptopropylthiomethyl)methane, bis(2,3-dimercaptopropyl)sulfide, 2,5-dimercaptomethyl-1,4-dithiane, 2,5-dimercapto-1,4-dithiane, 2,5-dimercaptomethyl-2,5-dimethl-1,4-dithiane and esters of thioglycolic acid and mercaptopropionic acid thereof, hydroxymethyl sulfide bis(2-mercaptoacetate), hydroxymethyl sulfide bis(3-mercaptopropionate), hydroxyethyl sulfide bis(2-mercaptoacetate), hydroxyethyl sulfide bis(3-mercaptopropionate), hydroxymethyl disulfide bis(2-mercaptoacetate), hydroxymethyl disulfide bis(3-mercaptopropionate), hydroxyethyl disulfide bis(2-mercaptoacetate), hydroxyethyl disulfide bis(3-mercaptopropionate), 2-mercaptoethyl ether bis(2-mercaptoacetate), 2-mercaptoethyl ether bis(3-mercaptopropionate), thioglycolic acid bis(2-mercaptoethyl ester), thiodipropionic acid bis(2-mercaptoethyl ester), dithiodiglycolic acid bis(2-mercaptoethyl ester), dithiodipropionic acid bis(2-mercaptoethyl ester), 1,1,3,3-tetrakis(mercaptomethylthio)propane, 1,1,2,2-tetrakis(mercaptomethylthio)ethane, 4,6-bis(mercaptomethylthio)-1,3-dithiane, tris(mercaptomethylthio)methane, and tris(mercaptoethylthio)methane; aromatic polythiol compounds such as 1,2-dimercaptobenzene, 1,3-dimercaptobenzene, 1,4-dimercaptobenzene, 1,2-bis(mercaptomethyl)benzene, 1,3-bis(mercaptomethyl)benzene, 1,4-bis(mercaptomethyl)benzene, 1,2-bis(mercaptoethyl)benzene, 1,3-bis(mercaptoethyl)benzene, 1,4-bis(mercaptoethyl)benzene, 1,3,5-trimercaptobenzene, 1,3,5-tris(mercaptomethyl)benzene, 1,3,5-tris(mercaptomethyleneoxy)benzene, 1,3,5-tris(mercaptoethyleneoxy)benzene, 2,5-toluenedithiol, 3,4-toluenedithiol, 1,5-naphthalenedithiol, and 2,6-naphthalenedithiol; and heterocyclic polythiol compounds such as 2-methylamino-4,6-dithiol-sym-triazine, 3,4-thiophene dithiol, bismuthiol, 2,5-bis(mercaptomethyl)-1,4-dithiane, 4,6-bis(mercaptomethylthio)-1,3-dithiane, and 2-(2,2-bis(mercaptomethylthio)ethyl)-1,3-dithietane, and at least one selected from these can be used.

The polysulfide can be obtained by the ring-opening polymerization method of a polyepithio compound or a polythietane compound or the like as a monomer. The composition for optical elements can contain monomers constituting these polymers.

The polyepithio compound can be used without particular limitation, and for example, those described in Japanese Patent No. 6216383 can be used.

As the polythietane compound, a metal-containing thietane compound or a non-metal thietane compound can be used. Specifically, for example, those described in Japanese Patent No. 6216383 can be used.

The polycarbonate can be obtained by a reaction of an alcohol with phosgene, a method of reacting an alcohol with chloroformate, or a transesterification reaction of a carbonic acid diester compound, but it is also possible to use a commercially available polycarbonate resin which is generally available. As commercially available products, Panlite series manufactured by Teijin Chemicals Ltd., and the like can be used.

The poly(meth)acrylate can be used without particular limitation, and for example, those described in Japanese Patent No. 6216383 can be used.

The polyolefin can be used without particular limitation, and for example, specific examples described in Japanese Patent No. 6216383, a cyclic polyolefin, a polymerization reaction of an olefin, and a method of producing a polyolefin can be used.

The polyallyl is produced by polymerizing at least one allyl group-containing monomer selected from allyl group-containing monomers in the presence of a known radical generating polymerization catalyst.

As the allyl group-containing monomer, allyl diglycol carbonate and diallyl phthalate are generally commercially available, and these can be suitably used.

The polyurethane urea is a reaction product obtained from a polyurethane prepolymer and a diamine curing agent, and one sold by PPG Industries, Inc. as trademark TRIVEX is a representative example. The polyurethane urea is a highly transparent material, and can be suitably used.

The polyene-polythiol polymer is a polymer product obtained by addition polymerization and ethylene chain polymerization and derived from a polyene compound having two or more ethylenic functional groups in one molecule and a polythiol compound having two or more thiol groups in one molecule.

As the polyene compound in the polyene-polythiol polymer, for example, those described in Japanese Patent No. 6216383 can be used.

The ring-opened metathesis polymer is a polymer obtained by the ring-opening polymerization of cyclic olefins using a catalyst. As the cyclic olefins that can be subjected to ring-opening polymerization, for example, those described in Japanese Patent No. 6216383 can be used.

The polyester is condensation polymerized in the presence of a known polyester production catalyst such as a Lewis acid catalyst typified by an antimony or germanium compound, an organic acid, or an inorganic acid. Specifically, the polyester refers to those composed of one or two or more selected from polyvalent carboxylic acids containing dicarboxylic acids and ester-forming derivatives thereof and one or two or more selected from polyhydric alcohols containing glycols, those composed of hydroxycarboxylic acids and ester-forming derivatives thereof, or those composed of cyclic esters.

As the dicarboxylic acid and the glycol, for example, those described in Japanese Patent No. 6216383 can be used.

As the polyester, for example, those described in Japanese Patent No. 6216383 can be used.

The epoxy resin is a polymer obtained by the ring-opening polymerization of an epoxy compound, and as the epoxy compound, for example, those described in Japanese Patent No. 6216383 can be used.

(Additive)

The optical element of the disclosure may contain an additive as a component other than the above components.

Examples of the additive include a polymerization catalyst, an internal mold release agent, a dye, and an ultraviolet absorber. In the disclosure, in a case where a polyurethane or a polythiourethane is obtained, the polymerization catalyst may be used, or is not necessarily used.

Examples of the internal mold release agent include an acidic phosphoric acid ester. Examples of the acidic phosphoric acid ester include a phosphoric monoester and a phosphoric diester, and each of these can be used singly or in a mixture of two or more kinds.

Examples of the ultraviolet absorber include benzophenone-based ultraviolet absorbers such as 2,2'-dihydroxy-4-methoxybenzophenone, 2-hydroxy-4-acryloyloxybenzophenone, 2-hydroxy-4-acryloyloxy-5-tert-butylbenzophenone, and 2-hydroxy-4-acryloyloxy-2',4'-dichlorobenzophenone, triazine-based ultraviolet absorbers such as 2-[4-[(2-hydroxy-3-dodecyloxypropyl)oxy]-2-hydroxyphenyl]4,6-bis(2,4-dimethylphenyl)-1,3,5-triazine, 2-[4-(2-hydroxy-3-tridecyloxypropyl)oxy]-2-hydroxyphenyl]-4,6-bis(2,4 dimethylphenyl)-1,3,5-triazine, 2-[4-[(2-hydroxy-3-(2'-ethyl)hexyl)oxy]-2-hydroxyphenyl]-4,6-bis(2,4-dimethylphenyl)-1,3,5-triazine, 2,4-bis(2-hydroxy-4-butyloxyphenyl)-6-(2,4-bis-butyloxyphenyl)-1,3,5-triazine, and 2-(2-hydroxy-4-[1-octyloxycarbonylethoxy]phenyl)-4,6-bis(4-phenylphenyl)-1,3,5-triazine, benzotriazole-based ultraviolet absorbers such as 2-(2H-benzotriazole-2-yl)-4-methylphenol, 2-(2H-benzotriazole-2-yl)-4-tert-octylphenol, 2-(2H-benzotriazole-2-yl)-4,6-bis(1-methyl-1-phenylethyl)phenol, 2-(2H-benzotriazole-2-yl)-4,6-di-tert-pentylphenol, 2-(5-chloro-2H-benzotriazole-2-yl)-4-methyl-6-tert-butylphenol, 2-(5-chloro-2H-benzotriazole-2-yl)-2,4-tert-butylphenol, and 2,2'-methylenebis[6-(2H-benzotriazole-2-yl)-4-(1,1,3,3-tetramethylbutyl)phenol], and benzotriazole-based ultraviolet absorbers such as 2-(2H-benzotriazole-2-yl)-4-tert-octylphenol and 2-(5-chloro-2H-benzotriazole-2-yl)-4-methyl-6-tert-butylphenol are preferable. These ultraviolet absorbers may be used singly or in combination of two or more kinds thereof.

As the ultraviolet absorber, a commercially available product may be used. Examples of the commercially available product include Tinuvin 326 (manufactured by BASF Japan Ltd.) and Viosorb 583 (manufactured by Kyodo Chemical Company Limited).

The optical element of the disclosure may contain a color tone adjusting agent.

The color tone adjusting agent to be used may be a color tone adjusting agent having an absorption band in an orange to yellow wavelength region in a visible light region and having a function of regulating the hue of an optical element containing a polymer.

As the color tone adjusting agent, a bluing agent may be used. The bluing agent to be used may be a bluing

agent having an absorption band in an orange to yellow wavelength range in a visible light region and having a function of regulating the hue of an optical element made of a resin material. The bluing agent may contain a substance exhibiting blue to purple.

«Eyeglass lens»

The eyeglass lens of the disclosure includes the optical element of the disclosure.

The eyeglass lens of the disclosure includes the optical element of the disclosure, and thereby the eyeglass lens can regulate the autonomic nerves.

(Luminous transmittance)

From the viewpoint of visibility, the eyeglass lens of the disclosure preferably has a luminous transmittance of 8% or more, more preferably 20% or more, and still more preferably 40% or more.

The eyeglass lens of the disclosure may have a luminous transmittance of 90% or less, 85% or less, or 80% or less.

The eyeglass lens of the disclosure preferably also has a luminous transmittance of from 8% to 90%.

The luminous transmittance is measured in accordance with JIS T 7333: 2018.

The luminous transmittance can be measured using an eyeglass lens having a total thickness of 2 mm using a spectrophotometer (for example, CM-5 manufactured by Konica Minolta, Inc.).

<Composition for eyeglass lenses>

The eyeglass lens can be produced using, for example, a composition for eyeglass lenses described below.

The composition for eyeglass lenses contains a first colorant having a local maximum absorption wavelength b present within a range of from 450 nm to 540 nm in a spectrum measured in accordance with CIE 1976; and a second colorant having a local maximum absorption wavelength a present within a range of from more than 540 nm to 630 nm in a spectrum measured in accordance with CIE 1976, and a sum of a transmittance at the local maximum absorption wavelength a and a transmittance at the local maximum absorption wavelength b is from 20% to 120%.

The composition for eyeglass lenses may contain the above-mentioned polymer or the above-mentioned monomer, and may contain an additive such as an ultraviolet absorber.

The content of the colorant is preferably from 0.0001 parts by mass to 0.008 parts by mass, more preferably from 0.0001 parts by mass to 0.007 parts by mass, and still more preferably from 0.0002 parts by mass to 0.006 parts by mass with respect to 100 parts by mass of the total of the polymer and the monomer.

The content of the colorant means the total content of all colorants contained in the composition for eyeglass lenses.

In the disclosure, the composition for eyeglass lenses can be obtained by mixing the above components by a predetermined method.

The mixing order and mixing method and the like of the components in the composition are not particularly limited, and the components can be mixed by a known method. Examples of the known method include a method in which a masterbatch containing a predetermined amount of an additive is prepared, and the masterbatch is dispersed and dissolved in a solvent. For example, in the case of a polyurethane resin, examples thereof include a method in which an additive is dispersed and dissolved in a polyisocyanate compound to prepare a masterbatch.

<Aspect of eyeglass lens>

Suitable examples of an aspect of the eyeglass lens of the disclosure include sports eyeglasses, a swimming goggle, a goggle, a light-shielding lens, and sunglasses from the viewpoint of regulating the autonomic nerves.

Examples of the aspect of the eyeglass lens of the disclosure include an eyeglass lens including a functional layer, and an eyeglass lens including a functional layer and a coating layer.

Examples of the substrate include a lens substrate.

Examples of the coating layer include a primer layer, a hard coat layer, an antireflection layer, an antifogging coat layer, an antifouling layer, and a water-repellent layer. Each of these coating layers can be used singly, or a plurality of coating layers can be used in a multilayered manner. In a case where the coating layer is applied to each of both surfaces, the same coating layer may be applied to both the surfaces, or different coating layers may be applied to the surfaces.

For example, a molded body (for example, a lens substrate) may be prepared using a composition for eyeglass lenses not containing a colorant, followed by immersing the molded body in a dispersion liquid obtained by dispersing

the colorant in water or a solvent to impregnate the molded body with the colorant, and drying the molded body impregnated with the colorant. The eyeglass lens can be prepared using the molded body thus obtained.

After the eyeglass lens is prepared, the eyeglass lens can also be impregnated with the porphyrin-based compound. In addition, an eyeglass lens including a lens substrate and a coating layer laminated if necessary can also be immersed in a dispersion liquid containing a colorant to impregnate the lens with the colorant.

The impregnation amount of the colorant may be regulated to a desired impregnation amount by the concentration of the colorant in the dispersion liquid, the temperature of the dispersion liquid, and a time for immersing the molded body and the eyeglass lens and the like. As the concentration and the temperature are higher, and the immersion time is longer, the impregnation amount is greater. In a case where the impregnation amount is more precisely regulated, the immersion may be repeated a plurality of times under a condition that the impregnation amount is small.

A colorant-containing coating layer may be formed on an eyeglass lens such as a plastic lens by using a composition for eyeglass lenses containing a colorant as a coating material.

The disclosure is not limited to the above-described embodiments, and various aspects can be taken as long as the effects of the present invention are not impaired.

Specific examples of the coating layer include an antireflection layer, a primer layer, a hard coat layer, an antifogging coat layer, an antifouling layer, and a water-repellent layer.

Each of these coating layers can be used singly, or a plurality of coating layers can be used in a multilayered manner. In a case where the coating layer is applied to each of both surfaces, the same coating layer may be applied to both the surfaces, or different coating layers may be applied to the surfaces.

These coating layers may contain a colorant, an infrared absorber, a light stabilizer, an antioxidant, a dye, a pigment, a photochromic dye, a photochromic pigment, an antistatic agent, and other known additives for enhancing the performance of lenses, and the like used in the disclosure.

For the layer to be coated by coating, various leveling agents intended to improve coatability may be used.

The eyeglass lens of the disclosure preferably further includes an antireflection layer on its surface.

Examples of the antireflection layer include an inorganic antireflection layer and an organic antireflection layer.

The inorganic antireflection layer is formed using an inorganic oxide such as $SiO_2$ or $TiO_2$ by a dry method such as a vacuum vapor deposition method, a sputtering method, an ion plating method, an ion beam assist method, or a CVD method.

The organic antireflection layer is formed by a wet process using a composition containing an organosilicon compound and silica-based fine particles having an internal cavity.

The antireflection layer may be formed on the hard coat layer if necessary.

The antireflection layer may be a multilayer or a single layer.

From the viewpoint of effectively exhibiting an antireflection function, the antireflection layer is preferably a multilayer, and in this case, it is preferable to alternately laminate a low refractive index layer and a high refractive index layer. A difference in refractive index between the low refractive index layer and the high refractive index layer is preferably 0.1 or more.

Examples of the high refractive index layer include layers made of ZnO, $TiO_2$, $CeO_2$, $Sb2O_5$, $SnO_2$, $ZrO_2$, and $Ta_2O_5$ and the like, and examples of the low refractive index layer include a layer made of $SiO_2$.

In a case where the antireflection layer is used as the single layer, the refractive index of the antireflection layer is preferably lower than the refractive index of the hard coat layer by at least 0.1 or more.

An antifogging coat layer, an antifouling layer, and a water-repellent layer and the like may be formed on the antireflection layer if necessary. A method of forming the antifogging coat layer, the antifouling layer, and the water-repellent layer and the like is not particularly limited, and conventionally known methods can be applied.

The primer layer is usually formed between the hard coat layer and the lens substrate. The primer layer is a coating layer intended to improve adhesion between the hard coat layer formed thereon and the lens substrate. The primer layer may be capable of improving impact resistance.

The primer layer may have high adhesion to the obtained lens substrate. For forming the primer layer, for example, a primer composition containing an urethane-based resin, an epoxy-based resin, a polyester-based resin, a melamine-based resin, or polyvinyl acetal as a main component can be used.

In the preparation of the primer composition, a solvent is not necessarily used, but a solvent that does not affect the lens substrate may be used for the purpose of regulating the viscosity of the composition.

The primer layer can be formed by either a coating method or a dry method.

In a case where the coating method is used, the primer composition is applied to the lens substrate by a known coating method such as spin coating or dip coating, and then solidified to form the primer layer.

In a case where the dry method is used, the primer layer is formed by a known dry method such as a CVD method or a vacuum vapor deposition method. In forming the primer layer, the surface of the lens substrate may be subjected to a pretreatment such as an alkali treatment, a plasma treatment, or an ultraviolet treatment if necessary for the purpose of improving adhesion.

The hard coat layer is a coating layer intended to impart functions such as scratch resistance, abrasion resistance, moisture resistance, hot water resistance, heat resistance, and weather resistance to the surface of the lens.

In the formation of the hard coat layer, a hard coat composition containing an organic silicon compound having curability and one or more kinds of oxide fine particles containing an element selected from the element group of Si, Al, Sn, Sb, Ta, Ce, La, Fe, Zn, W, Zr, In, and Ti may be used, and a hard coat composition containing an organic silicon compound having curability and one or more kinds of fine particles of a composite oxide containing two or more kinds of elements selected from the element group of Si, Al, Sn, Sb, Ta, Ce, La, Fe, Zn, W, Zr, In, and Ti may be used.

In addition to the above components, the hard coat composition preferably contains at least one selected from the group consisting of amines, amino acids, metal acetylacetonate complexes, metal organic acid salts, perchloric acids, salts of perchloric acids, acids, metal chlorides, and polyfunctional epoxy compounds.

The hard coat composition may contain a solvent that does not affect the lens substrate, or does not necessarily contain the solvent.

The hard coat layer is usually formed by applying a hard coat composition by a known coating method such as spin coating or dip coating, and then curing the hard coat composition. Examples of the curing method include irradiation with energy rays such as ultraviolet rays and visible rays, and thermal curing. From the viewpoint of suppressing the occurrence of interference fringes, a difference in refractive index between the hard coat layer and the lens substrate is preferably within a range of ±0.1.

<<Autonomic nerve regulation method>>

The autonomic nerve regulation method of the disclosure is a method of regulating autonomic nerves through an optical element in which Stim (ipRGC) represented by the following Formula 1 is from 10.0 to 68.0.

The autonomic nerve regulation method of the disclosure includes suppressing an increase in a heart rate, LF/HF, and pNN 50 through an optical element in which Stim (ipRGC) represented by the following Formula 1 is from 10.0 to 68.0.

$$\mathrm{Stim(ipRGC)} = \frac{\int_{390nm}^{780nm}\{RI_{D65}(\lambda)\cdot\tau(\lambda)\cdot S_{mel}(\lambda)\}d\lambda}{\int_{390nm}^{780nm}\{RI_{D65}(\lambda)\cdot S_{mel}(\lambda)\}d\lambda} \qquad \text{Formula 1}$$

In Formula 1, $RI_{D65}$ (λ) is a spectral irradiance of illuminant D65, τ (λ) is a spectral transmittance of an optical element, and $S_{mel}$ (λ) is a sensitivity function of ipRGCs obtained by converting a sensitivity function of ipRGCs described in CIE S/026E: 2018 such that a maximum sensitivity thereof is identical to a maximum sensitivity of a z bar (λ) in CIE 1931 color-matching functions.

The inventors have found that there is a possibility that autonomic nerves can be affected by controlling light incident on a photoreceptor. In particular, the inventors have found that a heart rate, HF/LF, and pNN 50 are affected. As a result of further studies based on the above findings, the inventors have found that the autonomic nerves can be regulated by the autonomic nerve regulation method of the disclosure including the above configuration.

By using the autonomic nerve regulation method of the disclosure, for example, in the case of taking a walk or jogging outdoors while wearing the above-described eyeglass lens, or in the case where a headlight of a vehicle enters the eyes at night, a sudden increase in a heart rate due to light stimulation can be expected to be suppressed to reduce a burden on the body.

As described above, the autonomic nerve regulation method of the disclosure may be used for actions other than medical actions.

The autonomic nerve regulation method of the disclosure can also improve, for example, disorder caused by the disturbance of balance of the autonomic nerves.

Examples of such disorder include excessive vagal reflex, sympathetic nervous tone, and severe palpitation.

The autonomic nerve regulation method of the disclosure includes suppressing an increase in a heart rate due to light stimulation through the optical element in which Stim (ipRGC) represented by the above Formula 1 is from 10.0 to 68.0.

The details of the specific aspect, preferable aspect, preferable range of Stim (ipRGC), and preferable range of Stim (L) and the like of the optical element used in the autonomic nerve regulation method of the disclosure are the same as the details of the specific aspect, preferable aspect, preferable range of Stim (ipRGC), and preferable range of Stim (L) and the like of the optical element described in the section of <Optical element> described above.

In the autonomic nerve regulation method of the disclosure, other means may further be used in addition to the optical element in which Stim (ipRGC) represented by Formula 1 is from 10.0 to 68.0.

Examples of the other means include outdoor exercise such as jogging. The exercise such as jogging may rapidly increase the heart rate particularly immediately after the start. However, by controlling light stimulation such as sunlight through the optical element in which Stim (ipRGC) represented by Formula 1 is from 10.0 to 68.0, a rapid increase in the heart rate can be suppressed, and therefore the burden on the body can be reduced.
The use of the optical element of the disclosure may be use of the optical element in an optical product for regulating autonomic nerves.
The optical element of the disclosure may be an optical element for use in the optical product for regulating the autonomic nerves.

<Method of measuring heart rate variability>

In the autonomic nerve regulation method of the disclosure, heart rate variability is preferably measured using at least one selected from the group consisting of a change value of a heart rate by electrocardiography measurement, a ratio (LF/HF) of a low-frequency component LF to a high-frequency component HF of the heart rate, and pNN 50.
In the autonomic nerve regulation method of the disclosure, at least one selected from the group consisting of the heart rate, the ratio (LF/HF) of the low-frequency component LF to the high-frequency component HF of the heart rate, and pNN 50 is preferably regulated.
The details of LF/HF, heart rate (bpm), and pNN 50 (%) are as follows.

[LF/HF]

LF/HF is a ratio of power between LF and HF. This value represents, for example, the overall balance between sympathetic and parasympathetic nerves.
In a case where the numerical value is high, the sympathetic nerve is dominant, and in a case where the numerical value is low, the parasympathetic nerve is dominant.

[LF (Low frequency)]

LF ($ms^2$) is a power spectrum in a frequency band of 0.004 Hz to 0.150 Hz (that is, a low frequency). This value reflects both the sympathetic nerve (for example, vasomotor sympathetic nerve) and the parasympathetic nerve activities.
The influence of the parasympathetic nerve on the frequency band appears in LF while taking deep breath in which the respiration rate is 9 times (frequency: 0.15 Hz) or less per minute. Therefore, an LF value is high in a case where a subject is slowly and regularly breathing in a relaxed state. This means increased parasympathetic nerve activity rather than increased sympathetic nerve activity.

[HF (High frequency)]

HF ($ms^2$) is a power spectrum in a frequency band of 0.15 Hz to 0.40 Hz (that is, high frequency). This value reflects parasympathetic nerve (for example, vagus nerve) activity.
The heart rate increases in a case of inhaling and decreases in a case of exhaling. Slow, regular breathing increases the amplitude of an HF peak in the power spectrum.

[Heart rate (bpm)]

The heart rate is the number of beats per minute.

[pNN 50 (%)]

pNN 50 (percent of difference between adjacent normal RR intervals greater than 50 ms) is the percentage of heartbeats where a difference between consecutive adjacent RR intervals is greater than 50 ms, and is an indicator of vagal tone strength.

<Evaluation method for optical element>

An evaluation method for an optical element of the disclosure is an evaluation method for an optical element for regulating autonomic nerves, the method including:

a preparation step of preparing an optical element in which Stim (ipRGC) represented by the following Formula 1

is from 10.0 to 68.0; and

an evaluation step of carrying out evaluation by measuring a heart rate variability of a subject before and after irradiating the subject with artificial sunlight through the optical element for a predetermined time in a dark place with illuminance of 5 lux or less.

$$\mathrm{Stim(ipRGC)} = \frac{\int_{390nm}^{780nm} \{RI_{D65}(\lambda) \cdot \tau(\lambda) \cdot S_{mel}(\lambda)\} d\lambda}{\int_{390nm}^{780nm} \{RI_{D65}(\lambda) \cdot S_{mel}(\lambda)\} d\lambda} \qquad \text{Formula 1}$$

In Formula 1, $RI_{D65}$ (λ) is a spectral irradiance of illuminant D65, τ (λ) is a spectral transmittance of an optical element, and $S_{mel}$ (λ) is a sensitivity function of ipRGCs obtained by converting a sensitivity function of ipRGCs described in CIE S/026E: 2018 such that a maximum sensitivity thereof is identical to a maximum sensitivity of a z bar (λ) in CIE 1931 color-matching functions.

(Preparation step)

The preparation step is a step of preparing an optical element in which Stim (ipRGC) is from 10.0 to 68.0.

The optical element may be produced and prepared, or a commercially available product may be obtained and prepared.

Examples of the optical element include an eyeglass lens, a window of an automobile and the like, an illumination lamp, a film used for the illumination lamp, and a filter used for a digital device.

Among the above, an eyeglass lens is preferable as the optical element.

(Evaluation step)

The evaluation step is a step of carrying out evaluation by measuring a heart rate variability of a subject before and after irradiating the subject with artificial sunlight through the optical element for a predetermined time in a dark place with illuminance of 5 lux or less.

In the evaluation method for an optical element of the disclosure, the evaluation step preferably includes regulating autonomic nerves by comparing a heart rate variability of a subject in a dark place with illuminance of 5 lux or less in a state of not using the optical element and not irradiating the subject with artificial sunlight, with a heart rate variability of the subject before and after irradiating the subject with artificial sunlight through the optical element for a predetermined time in a dark place with illuminance of 5 lux or less.

Specifically, the evaluation step can be performed, for example, as follows.

First, the subject is allowed to enter a dark room with illuminance of 5 lux or less, and is allowed to rest for 5 to 10 minutes to measure the heart rate.

Thereafter, an eyeglass lens is worn on the subject, and light irradiation is performed for 5 minutes in a state where a light irradiation port of the artificial sunlight lamp and the subject face each other, to measure the heart rate. The illuminance of the artificial sunlight may be, for example, from 1000 lux to 5000 lux. In a state where the light irradiation port of the artificial sunlight lamp and the subject face each other, a distance between the light irradiation port of the artificial sunlight lamp and the subject may be, for example, 1 m.

A specific aspect of the method of measuring heart rate variability is the same as the specific aspect described in <Method of measuring heart rate variability>.

EXAMPLES

Hereinafter, an embodiment of the disclosure will be specifically described with reference to Examples, but the disclosure is not limited to these Examples.

[Production of eyeglass lenses 1 to 7]

(Preparation of masterbatch)

0.050 g of each of colorants of kinds shown in Table 1 was dissolved in 100.0 g of a composition containing 2,5(6)-bis(isocyanatomethyl)-bicyclo[2.2.1]heptane, to prepare a masterbatch in which the colorant was dissolved.

(Production of eyeglass lens 1)

Into a sufficiently dried flask, 0.020 g of dimethyltin (IV) dichloride, 0.10 g of an internal mold release agent for MR (manufactured by Mitsui Chemicals, Inc.), 1.50 g of Viosorb 583 (ultraviolet absorber, 2-(2'-hydroxy-5'-t-octylphenyl)benzotriazole, manufactured by Kyodo Chemical Company Limited), and 40.6 g of a composition containing 2,5(6)-bis(isocyanatomethyl)-bicyclo[2.2.1]heptane were charged to prepare a mixed liquid (1).

Subsequently, 10.0 g of the masterbatch in which the colorant was dissolved as shown in Table 1 was added to the mixed liquid (1) to obtain a mixed liquid (2). The mixed liquid (2) was stirred at 25°C for 1 hour to completely dissolve the components, thereby obtaining a prepared liquid. Thereafter, 23.9 g of a composition containing pentaerythritol tetrakis(3-mercaptopropionate) and 25.5 g of a composition containing 4-mercaptomethyl-1,8-dimercapto-3,6-dithiaoctane were added to the prepared liquid, and the obtained liquid was stirred at 25°C for 30 minutes to prepare a uniform solution.

Table 1 shows the contents of the colorants in the uniform solution.

This solution was defoamed at 400 Pa for 1 hour, filtered with a 1 μm PTFE (polytetrafluoroethylene) filter, and then injected into a 4C plano glass mold having a center thickness of 2 mm and a diameter of 77 mm.

The glass mold was heated from 25°C to 120°C over 21 hours. Thereafter, the glass mold was cooled to room temperature, and a plano lens was removed from the glass mold. The obtained plano lens was further annealed at 120°C for 2 hours. As a result, a molded body (that is, eyeglass lens 1) was obtained.

(Production of eyeglass lens 2, eyeglass lens 3, eyeglass lens 5, and eyeglass lens 7)

Molded bodies (that is, eyeglass lens 2, eyeglass lens 3, eyeglass lens 5, and eyeglass lens 7) were obtained in the same manner as in Example 1 except that the kinds and contents of the colorants in the uniform solution were changed as shown in Table 1.

(Production of eyeglass lens 4 and eyeglass lens 6)

Molded bodies (that is, eyeglass lens 4 and eyeglass lens 6) were obtained in the same manner as in Example 1 except that 1.0 g of Tinuvin 326 (ultraviolet absorber, 2-(2'-hydroxy-3'-tert-butyl-5'-methylphenyl)-5-chlorobenzotriazole, manufactured by BASF Japan Ltd.) was used instead of 1.50 g of Viosorb 583, and the kinds and contents of the colorants in the uniform solution were changed as shown in Table 1.

<Examples 1 to 5, Comparative Example 1, and Comparative Example 2>

[Measurement of heartbeats]

In each of Examples and Comparative Examples, heartbeats were measured by the following method.

First, a subject in his twenties was allowed to enter a darkroom with illuminance of 5 lux or less, and was allowed to rest for 5 minutes, to measure the heart rate.

Thereafter, the eyeglass lens described in Table 1 is worn on the subject, and light irradiation was performed for 5 minutes in a state where a light irradiation port of artificial sunlight lamp (illuminant D65) and the subject faced each other. The timing of measuring the heart rate is 5 minutes before the start of light irradiation to immediately before the start of light irradiation (for 5 minutes), and immediately after the start of light irradiation to 5 minutes after the start of light irradiation (for 5 minutes). An average heart rate for 5 minutes was calculated for each case. The illuminance of the artificial sunlight was 1000 lux to 5000 lux. In a state where the light irradiation port of the artificial sunlight lamp and the subject faced each other, a distance between the light irradiation port of the artificial sunlight lamp and the subject was 1 m.

LF/HF, a heart rate (bpm) and pNN 50 (%) were measured by SilmeeTM Bar type Lite (manufactured by TDK Corporation).

For each of LF/HF, the heart rate (bpm), and pNN 50 (%), an average for 5 minutes of light irradiation, and an increase/decrease amount from 5 minutes of rest to 5 minutes of light irradiation were measured.

Detailed data such as transmittances of the eyeglass lens 1 to the eyeglass lens 7 were also measured.

The results are shown in Table 1.

[Assessment Criteria for heartbeats]

In a case where the increase/decrease amount of LF/HF (also referred to as ΔLF/HF) from 5 minutes of rest to 5 minutes of light irradiation is 0 or less, it means that sympathetic nerve activity is suppressed, whereby the heart

rate is considered to decrease.

In a case where the increase/decrease amount (also referred to as Δ heart rate) of the heart rate from 5 minutes of rest to 5 minutes of light irradiation is 0 or less, it means that the heart rate is low.

When the increase/decrease amount (also referred to as ΔpNN 50) of pNN 50 from 5 minutes of rest to 5 minutes of light irradiation is 0 or more, it means that parasympathetic nerve activity (for example, vagal nerve activity) is activated, whereby the heart rate is considered to decrease.

<Comparative Example 3>

Heartbeats were measured in the same manner as in the method of Example 1 except that no eyeglass lens was worn. The results are shown in Table 1.

In a case where at least the Δ heart rate among the ΔLF/HF, the Δ heart rate, and the ΔpNN 50 is 0 or less, the autonomic nerves were determined to be regulated.

Fig. 1 is a graph showing spectra measured in accordance with CIE 1976 for the eyeglass lenses of Examples 1 to 3.

Fig. 2 is a graph showing spectra measured in accordance with CIE 1976 for the eyeglass lenses of Example 4, Example 5, Comparative Example 1, and Comparative Example 2.

[Table 1]

| Examples | | Example 1 | Example 2 | Example 3 | Example 4 | Example 5 | Comparative Example 1 | Comparative Example 2 | Comparative Example 3 |
|---|---|---|---|---|---|---|---|---|---|
| Eyeglass lens | | 1 | 2 | 3 | 4 | 5 | 6 | 7 | - |
| Local maximum absorption wavelength of ultraviolet absorber [nm] | | 400 | 400 | 400 | 420 | 400 | 420 | 400 | - |
| Colorant 1 | Kind | UVY-1023 | UVY-1023 | UVY-1023 | UVY-1023 | UVY-1023 | FDB-003 | FDG-003 | - |
| | Mass ppm | 50 | 20 | 7 | 15 | 10.1 | 5.5 | 3.5 | - |
| Colorant 2 | Kind | - | - | FDB-003 | FDB-003 | PD-311S | FDG-003 | ABS 584 | - |
| | Mass ppm | - | - | 3 | 4.3 | 12.4 | 8 | 20 | - |
| Colorant 3 | Kind | - | - | FDG-002 | - | - | - | - | - |
| | Mass ppm | - | - | 10 | - | - | - | - | - |
| Local maximum absorption wavelength a [nm] | | 482 | 482 | 530 | 482 | 482 | - | - | - |
| Transmittance at local maximum absorption wavelength a [%] | | 2.1 | 19.6 | 33.4 | 28.8 | 40.5 | - | - | - |
| Local maximum absorption wavelength b [nm] | | 587 | 587 | 586 | 587 | 588 | 553 | 587 | - |
| Transmittance at local maximum absorption wavelength b [%] | | 53.9 | 73.2 | 83.6 | 77.0 | 23.7 | 55.4 | 10.2 | - |
| Value obtained by subtracting local maximum absorption wavelength a from local maximum absorption wavelength b [nm] | | 105 | 105 | 56 | 105 | 106 | - | - | - |
| Sum of transmittance at local maximum absorption wavelength a and transmittance at local maximum absorption wavelength b | | 56.1 | 92.8 | 117.0 | 105.8 | 64.2 | - | - | - |
| Luminous transmittance [%] | | 61.5 | 75.4 | 66.1 | 77.9 | 64.6 | 73.2 | 57.4 | - |
| Stim (L) | | 59.9 | 74.1 | 67.3 | 76.4 | 64.0 | 74.3 | 58.7 | 100.0 |
| Stim (ipRGC) | | 29.1 | 50.4 | 47.5 | 50.8 | 61.9 | 68.7 | 79.0 | 100.0 |
| Sum of Stim (L) and Stim (ipRGC) | | 89.0 | 124.5 | 114.7 | 127.2 | 125.9 | 143.0 | 137.7 | 200.0 |

(continued)

| Examples | | Example 1 | Example 2 | Example 3 | Example 4 | Example 5 | Comparative Example 1 | Comparative Example 2 | Comparative Example 3 |
|---|---|---|---|---|---|---|---|---|---|
| LF/HF | Average for 5 minutes of light irradiation | 2.0 | 2.7 | 1.4 | 3.0 | 7.8 | 2.1 | 3.2 | 3.2 |
| | Increase/decrease amount of LF/HF from 5 minutes of rest to 5 minutes of light irradiation (ΔLF/HF) | -1.7 | -0.3 | -0.6 | 0.3 | -4.3 | 0.4 | 0.5 | 1.6 |
| Heart rate [bpm] | Average for 5 minutes of light irradiation | 74.0 | 73.7 | 68.2 | 68.2 | 102.1 | 67.9 | 69.1 | 72.8 |
| | Increase/decrease amount of heart rate from 5 minutes of rest to 5 minutes of light irradiation (Δheart rate) | -2.3 | -3.4 | -0.7 | 0.0 | -1.5 | 3.3 | 2.4 | 4.7 |
| pNN 50 [%] | Average for 5 minutes of light irradiation | 28.6 | 28.7 | 36.7 | 37.2 | 1.4 | 34.1 | 30.1 | 26.8 |
| | Increase/decrease amount of pNN 50 from 5 minutes of rest to 5 minutes of light irradiation (ΔpNN 50) | 3.7 | 8.0 | 2.1 | 5.2 | -0.2 | -8.3 | -7.1 | -7.7 |

In Table 1, "-" means that the corresponding operation is not performed or the corresponding value does not exist.

The details of the colorants described in Table 1 are as follows.

UVY-1023: the above-described first colorant, manufactured by Yamamoto Chemicals, Inc.

FDB-003: the above-described first colorant, manufactured by Yamada Chemical Co., Ltd.

PD-311S: the above-described second colorant, manufactured by Yamamoto Chemicals, Inc.

FDG-003: the above-described second colorant, manufactured by Yamada Chemical Co., Ltd.

ABS 584: the above-described second colorant, manufactured by Luxottica

FDG-002: the above-described second colorant, manufactured by Yamada Chemical Co., Ltd.

As shown in Table 1, in the Examples using the autonomic nerve regulation method in which the autonomic nerves were regulated through the optical element (the above-described eyeglass lens) in which Stim (ipRGC) represented by Formula 1 was from 10.0 to 68.0, the Δ heart rate was 0 or less. Therefore, in the Examples, the increase in the heart rate could be suppressed by controlling light incident on a photoreceptor.

The eyeglass lens of each of the Examples is an eyeglass lens including a functional layer, wherein the functional layer has, in a spectrum measured in accordance with CIE 1976, (A) a local maximum absorption wavelength a present within a range of from 450 nm to 540 nm, and (B) a local maximum absorption wavelength b present within a range of from more than 540 nm to 630 nm, a transmittance at the local maximum absorption wavelength a is from 2% to 50%, a transmittance at the local maximum absorption wavelength b is from 10% to 90%, and a sum of the transmittance at the local maximum absorption wavelength a and the transmittance at the local maximum absorption wavelength b is from 20% to 120%.

Meanwhile, in Comparative Example 1 and Comparative Example 2 using the eyeglass lens in which Stim (ipRGC) was more than 68.0, the Δ heart rate was more than 0. Therefore, Comparative Example 1 and Comparative Example 2 could not suppress the increase in the heart rate.

In Comparative Example 3 in which the optical element was not used, the Δ heart rate was more than 0. Therefore, in Comparative Example 3, the increase in the heart rate could not be suppressed.

Among the Examples, Examples 1 to 3 in which the sum of Stim (ipRGC) and Stim (L) represented by Formula 2 was 125.0 or less showed that the heart rate was low in all of ΔLF/HF, Δ heart rate, and ΔpNN 50. Therefore, Examples 1 to 3 could more favorably suppress the increase in the heart rate.

The disclosure of Japanese Patent Application No. 2021-060675 filed on March 31, 2021 is incorporated herein by reference in its entirety.

All documents, patent applications, and technical standards described in this specification are incorporated herein by reference to the same extent as if each individual document, patent application, or technical standard was specifically and individually indicated to be incorporated by reference.

## Claims

1. An optical element, in which Stim (ipRGC) represented by the following Formula 1 is from 10.0 to 68.0:

$$\mathrm{Stim(ipRGC)} = \frac{\int_{390nm}^{780nm}\{RI_{D65}(\lambda)\cdot\tau(\lambda)\cdot S_{mel}(\lambda)\}d\lambda}{\int_{390nm}^{780nm}\{RI_{D65}(\lambda)\cdot S_{mel}(\lambda)\}d\lambda} \qquad \text{Formula 1}$$

wherein, in Formula 1, $RI_{D65}$ (λ) is a spectral irradiance of illuminant D65, τ (λ) is a spectral transmittance of the optical element, and $S_{mel}$ (λ) is a sensitivity function of ipRGCs obtained by converting a sensitivity function of ipRGCs described in CIE S/026E: 2018 such that a maximum sensitivity thereof is identical to a maximum sensitivity of a z bar (λ) in CIE 1931 color-matching functions.

2. The optical element according to claim 1, wherein a sum of the Stim (ipRGC) and Stim (L) represented by the following Formula 2 is from 20.0 to 135.0:

$$\mathrm{Stim}(L) = \frac{\int_{390nm}^{780nm}\{RI_{D65}(\lambda)\cdot\tau(\lambda)\cdot S_{L}(\lambda)\}d\lambda}{\int_{390nm}^{780nm}\{RI_{D65}(\lambda)\cdot S_{L}(\lambda)\}d\lambda} \qquad \text{Formula 2}$$

wherein, in Formula 2, $RI_{D65}$ (λ) is the spectral irradiance of illuminant D65, τ (λ) is the spectral transmittance of the optical element, and $S_L$ (λ) is a sensitivity function of L cones obtained by converting a sensitivity function of L cones described in CIE S/026E: 2018 such that a maximum sensitivity thereof is identical to a maximum sensitivity of an x bar (λ) in CIE 1931 color-matching functions.

**3.** An optical element, comprising a functional layer, wherein:

the functional layer has, in a spectrum measured in accordance with CIE 1976, (A) a local maximum absorption wavelength a that is present within a range of from 450 nm to 540 nm, and (B) a local maximum absorption wavelength b that is present within a range of from more than 540 nm to 630 nm,
a transmittance at the local maximum absorption wavelength a is from 2% to 50%,
a transmittance at the local maximum absorption wavelength b is from 10% to 90%, and
a sum of the transmittance at the local maximum absorption wavelength a and the transmittance at the local maximum absorption wavelength b is from 20% to 120%.

**4.** The optical element according to claim 3, wherein a value obtained by subtracting the local maximum absorption wavelength a from the local maximum absorption wavelength b is from 50 nm to 110 nm.

**5.** The optical element according to claim 3 or 4, wherein the functional layer contains at least one polymer selected from the group consisting of polyurethanes, polythiourethanes, polysulfides, polycarbonates, poly(meth)acrylates, and poly(thio)epoxides.

**6.** An eyeglass lens, comprising the optical element according to any one of claims 1 to 5.

**7.** The eyeglass lens according to claim 6, having a luminous transmittance of from 8% to 90%.

**8.** An autonomic nerve regulation method, comprising regulating autonomic nerves through an optical element, in which Stim (ipRGC) represented by the following Formula 1 is from 10.0 to 68.0:

$$\mathrm{Stim(ipRGC)} = \frac{\int_{390nm}^{780nm} \{RI_{D65}(\lambda) \cdot \tau(\lambda) \cdot S_{mel}(\lambda)\} d\lambda}{\int_{390nm}^{780nm} \{RI_{D65}(\lambda) \cdot S_{mel}(\lambda)\} d\lambda} \quad \text{Formula 1}$$

wherein, in Formula 1, $RI_{D65}$ (λ) is a spectral irradiance of illuminant D65, τ (λ) is a spectral transmittance of the optical element, and $S_{mel}$ (λ) is a sensitivity function of ipRGCs obtained by converting a sensitivity function of ipRGCs described in CIE S/026E: 2018 such that a maximum sensitivity thereof is identical to a maximum sensitivity of a z bar (λ) in CIE 1931 color-matching functions.

**9.** The autonomic nerve regulation method according to claim 8, wherein, in the optical element, a sum of the Stim (ipRGC) and Stim (L) represented by the following Formula 2 is from 20.0 to 135.0:

$$\mathrm{Stim}(L) = \frac{\int_{390nm}^{780nm} \{RI_{D65}(\lambda) \cdot \tau(\lambda) \cdot S_L(\lambda)\} d\lambda}{\int_{390nm}^{780nm} \{RI_{D65}(\lambda) \cdot S_L(\lambda)\} d\lambda} \quad \text{Formula 2}$$

wherein, in Formula 2, $RI_{D65}$ (λ) is the spectral irradiance of illuminant D65, τ (λ) is the spectral transmittance of the optical element, and $S_L$ (λ) is a sensitivity function of L cones obtained by converting a sensitivity function of L cones described in CIE S/026E: 2018 such that a maximum sensitivity thereof is identical to a maximum sensitivity of an x bar (λ) in CIE 1931 color-matching functions.

**10.** The autonomic nerve regulation method according to claim 8 or 9, comprising regulating at least one selected from the group consisting of a heart rate, a ratio (LF/HF) of a low-frequency component LF to a high-frequency component HF of the heart rate, and pNN 50.

**11.** An evaluation method for an optical element for regulating autonomic nerves, the method comprising:

a preparation step of preparing an optical element, in which Stim (ipRGC) represented by the following Formula 1 is from 10.0 to 68.0; and
an evaluation step of carrying out evaluation by measuring a heart rate variability of a subject before and after irradiating the subject with artificial sunlight through the optical element for a predetermined time in a dark place with illuminance of 5 lux or less:

$$\mathrm{Stim(ipRGC)} = \frac{\int_{390nm}^{780nm} \{RI_{D65}(\lambda) \cdot \tau(\lambda) \cdot S_{mel}(\lambda)\} d\lambda}{\int_{390nm}^{780nm} \{RI_{D65}(\lambda) \cdot S_{mel}(\lambda)\} d\lambda} \quad \text{Formula 1}$$

wherein, in Formula 1, $RI_{D65}$ (λ) is a spectral irradiance of illuminant D65, τ (λ) is a spectral transmittance of the optical element, and $S_{mel}$ (λ) is a sensitivity function of ipRGCs obtained by converting a sensitivity function of ipRGCs described in CIE S/026E: 2018 such that a maximum sensitivity thereof is identical to a maximum sensitivity of a z bar (λ) in CIE 1931 color-matching functions.

**12.** The evaluation method for an optical element according to claim 11, wherein the evaluation step includes regulating autonomic nerves by comparing a heart rate variability of the subject in the dark place with illuminance of 5 lux or less in a state of not using the optical element and not irradiating the subject with the artificial sunlight, with the heart rate variability of the subject before and after irradiating the subject with the artificial sunlight through the optical element for the predetermined time in the dark place with illuminance of 5 lux or less.

FIG.1

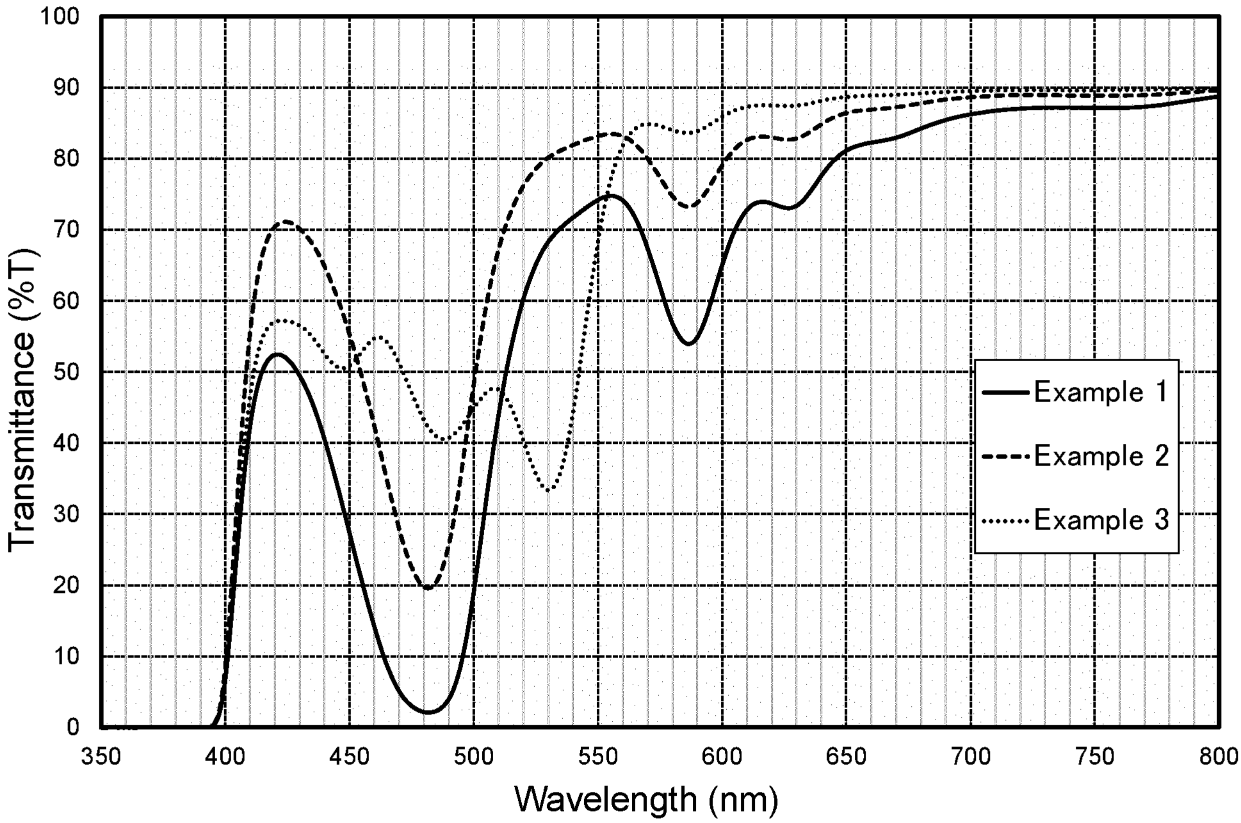
100
90
80
70
60
50
40
30
20
10
0
Transmittance (%T)
350
400
450
500
550
600
650
700
750
800
Wavelength (nm)
Example 1
Example 2
Example 3

FIG.2

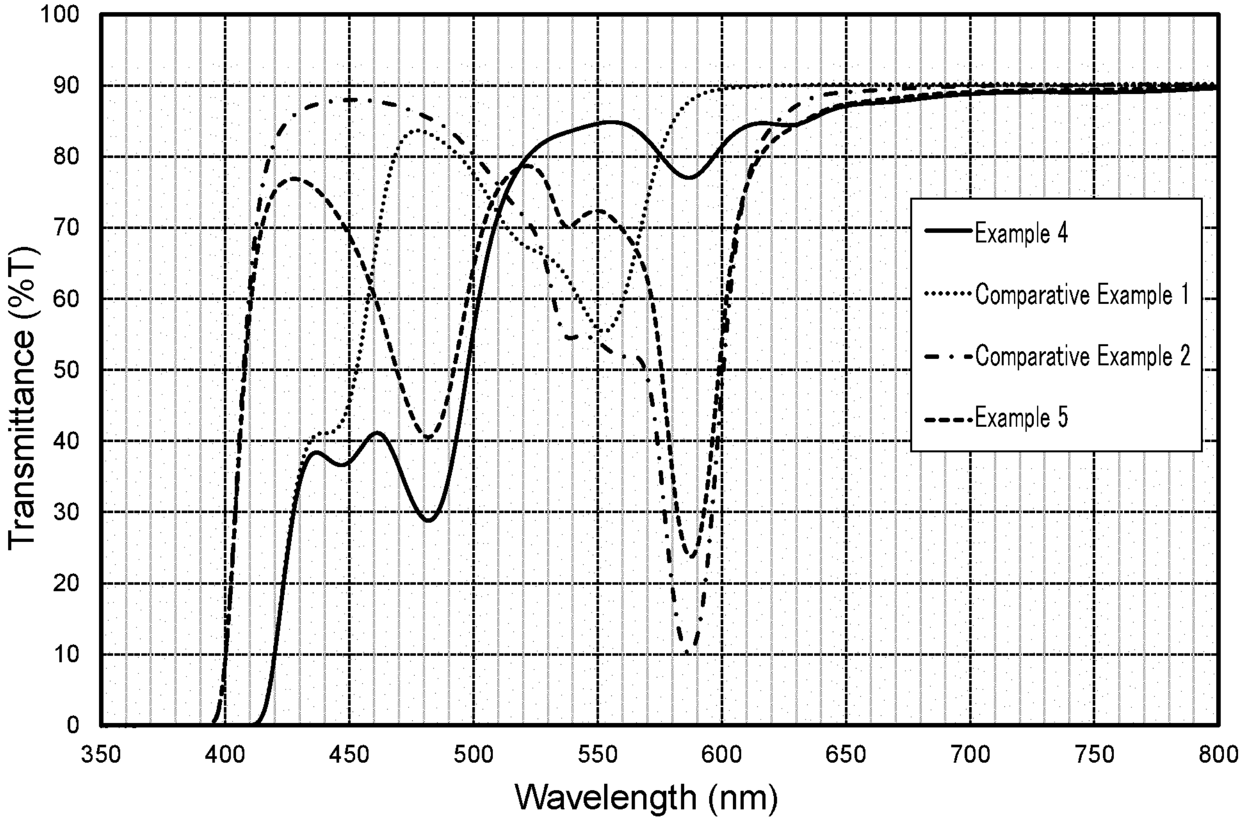
100
90
80
70
60
50
40
30
20
10
0
Transmittance (%T)
350
400
450
500
550
600
650
700
750
800
Wavelength (nm)
Example 4
Comparative Example 1
Comparative Example 2
Example 5

## INTERNATIONAL SEARCH REPORT

International application No. **PCT/JP2022/012027**

**A. CLASSIFICATION OF SUBJECT MATTER**

***G02C 7/10***(2006.01)i; ***A61B 5/0245***(2006.01)i; ***G02B 1/04***(2006.01)i; ***G02B 5/22***(2006.01)i
FI: G02C7/10; A61B5/0245 200; G02B1/04; G02B5/22

According to International Patent Classification (IPC) or to both national classification and IPC

**B. FIELDS SEARCHED**

Minimum documentation searched (classification system followed by classification symbols)

G02C7/10; A61B5/0245; G02B1/04; G02B5/22

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | US 2015/0192800 A1 (DIRK, Carl W.) 09 July 2015 (2015-07-09) claims, paragraphs [0003], [0016], [0022], examples 3-5, fig. 5-7 | 1-12 |
| Y | | 8-12 |
| X | JP 2020-530587 A (UNIVERSITY OF UTAH RESEARCH FOUNDATION) 22 October 2020 (2020-10-22) claims, paragraphs [0028], [0081]-[0082], [0117]-[0119], fig. 18, 32 | 1-7 |
| Y | | 8-12 |
| X | WO 2020/213716 A1 (MITSUI CHEMICALS, INC) 22 October 2020 (2020-10-22) claims, paragraphs [0006]-[0008], [0059], examples 1-2, fig. 1-2 | 1-7 |
| Y | | 8-12 |
| Y | JP 2020-532065 A (BRAINLIT AB) 05 November 2020 (2020-11-05) paragraphs [0008]-[0012], [0044]-[0045] | 8-12 |
| Y | JP 2019-535129 A (LUMILEDS LLC) 05 December 2019 (2019-12-05) paragraphs [0010]-[0011] | 8-12 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 May 2022** | **31 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/012027

C. DOCUMENTS CONSIDERED TO BE RELEVANT

| Category* | Citation of document, with indication, where appropriate, of the relevant passages | Relevant to claim No. |
|---|---|---|
| A | WO 2021/024962 A1 (MITSUI CHEMICALS, INC) 11 February 2021 (2021-02-11) entire text, all drawings | 1-12 |
| A | JP 2014-513818 A (ESSILOR INTERNATIONAL (COMPAGNIE GENERALE D'OPTIQUE)) 05 June 2014 (2014-06-05) entire text, all drawings | 1-12 |
| A | JP 2017-207626 A (ITO OPTICAL IND CO LTD) 24 November 2017 (2017-11-24) entire text, all drawings | 1-12 |

Form PCT/ISA/210 (second sheet) (January 2015)

**INTERNATIONAL SEARCH REPORT**
**Information on patent family members**

International application No.
**PCT/JP2022/012027**

| Patent document cited in search report | Publication date (day/month/year) | Patent family member(s) | Publication date (day/month/year) |
|---|---|---|---|
| US 2015/0192800 A1 | 09 July 2015 | WO 2014/011581 A2 | |
| JP 2020-530587 A | 22 October 2020 | WO 2019/032348 A1<br>claims, paragraphs [0059], [0112]-[0113], [0148]-[00150], fig. 18, 32<br>WO 2012/177296 A1<br>WO 2016/014713 A1<br>JP 2017-533449 A<br>JP 2020-190749 A<br>US 2017/0336545 A1<br>US 2014/0327967 A1<br>US 2014/0135570 A1<br>US 2019/0310405 A1<br>US 2020/0192011 A1<br>US 2014/0160569 A1<br>EP 2896423 A1<br>KR 10-2020-0053471 A<br>CN 111148482 A | |
| WO 2020/213716 A1 | 22 October 2020 | (Family: none) | |
| JP 2020-532065 A | 05 November 2020 | US 2020/0254274 A1<br>paragraphs [0012]-[0016], [0053]-[0054]<br>WO 2019/042933 A1<br>EP 3449975 A1<br>CN 111032153 A<br>KR 10-2020-0044875 A | |
| JP 2019-535129 A | 05 December 2019 | US 2018/0073689 A1<br>paragraphs [0010]-[0011]<br>US 2019/0219234 A1<br>WO 2018/049397 A1<br>TW 201821119 A<br>KR 10-2019-0047016 A<br>CN 110024141 A | |
| WO 2021/024962 A1 | 11 February 2021 | (Family: none) | |
| JP 2014-513818 A | 05 June 2014 | JP 2015-163879 A<br>JP 2016-130740 A<br>US 2013/0341523 A1<br>US 2014/0008543 A1<br>US 2016/0320302 A1<br>WO 2013/092377 A1<br>EP 2607884 A1<br>CN 104011532 A<br>KR 10-2014-0107307 A | |
| JP 2017-207626 A | 24 November 2017 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6216383 B
- JP 2021060675 A


### Non-patent literature cited in the description


- *Inorg. Chem.,* 1991, vol. 30, 239-245